# EUROPÄISCHE PATENTANMELDUNG

(11) **EP 3 906 923 A1**
(43) Veröffentlichungstag der Anmeldung: **10.11.2021**
(21) Anmeldenummer: 20173673.3
(22) Anmeldetag: 08.05.2020
(51) Int. Cl.: A61K 31/4355, A61K 31/4375, A61P 31/14, A61L 26/00, A61Q 17/00

(54) **ZUSAMMENSETZUNG UND VERFAHREN ZUR BEHANDLUNG VON COVID-19 MIT ISOCHINOLINALKALOID**

(71) Anmelder: Phytobiotics Futterzusatzstoffe GmbH, 65343 Eltville (DE)
(72) Erfinder: ROTH, Hermann, 65346 Eltville (DE)
(74) Vertreter: Richardt Patentanwälte PartG mbB

(57) **Zusammenfassung**

Die Erfindung betrifft ein Verfahren (300) zur akuten oder vorbeugenden Behandlung von Covid-19, beinhaltend:
- a) Erzeugung (302) eines Aerosols an einem Ort, wo das Aerosol von zumindest einem Menschen inhaliert werden kann, wobei das Aerosol aus Schwebeteilchen besteht, die zumindest ein Isochinolinalkaloid beinhalten; und/oder
- b) Behandlung (304) eines Objektes (400, 500, 600, 700) mit einer wässrigen Lösung, die zumindest ein Isochinolinalkaloid beinhaltet, oder mit einem Aerosol, das zumindest ein Isochinolinalkaloid beinhaltet, wobei das Objekt ein Gerät, Kleidungsstück oder Gegenstand ist, das für den Kontakt mit der menschlichen Haut bestimmt ist.

## Beschreibung

### Technisches Gebiet

Die vorliegenden Darstellungen betreffen Verfahren, Objekte und Zusammensetzungen zur vorbeugenden und akuten Behandlung der Atemwegserkrankung COVID-19.

### Stand der Technik

SARS-CoV-2 (Abk. für englisch severe acute respiratory syndrome coronavirus 2) ist ein im Januar 2020 in der chinesischen Stadt Wuhan, Provinz Hubei, neu identifiziertes Virus der Virusfamilie Coronaviridae. Das Virus verursacht die neue Atemwegserkrankung COVID-19 und war Auslöser der COVID-19-Pandemie. Das Virus wird hauptsächlich durch Tröpfcheninfektion, zum Teil aber auch durch Schmierinfektion übertragen.

Für die Krankheit COVID-19 gibt es bisher keine spezifische Behandlung, eine Therapie zielt darauf ab, die Symptome zu lindern. Es gibt erste Hinweise auf eine potentielle Wirksamkeit von Chloroquin. Aufgrund dessen hoher Toxizität ist die Verwendbarkeit dieses Mittels insbesondere zur vorbeugenden Behandlung und zur Behandlung von milden Verläufen jedoch fraglich. In der Publikation "In Vitro Antiviral Activity and Projection of Optimized Dosing Design of Hydroxychloroquine for the Treatment of Severe Acute Respiratory Syndrome Coronavirus 2 (SARS-CoV-2)" von Xueting Yao et al., erschienen in: Clinical Infectious Diseases. 9. März 2020, doi:10.1093/cid/ciaa237, PMID 32150618 (Vorabveröffentlichung) untersuchen die Autoren die Wirksamkeit von Hydroxychloroquin und dessen immunmodulierende Wirkung bei der Kontrolle des Zytokinsturms, der in der Spätphase bei kritisch kranken SARS-CoV-2-infizierten Patienten auftritt. Hydroxychloroquin erwies sich als wirksamer als Chloroquin zur Hemmung von SARS-CoV-2 in vitro. Allerdings tritt der Zytokinsturm nur bei wenigen Patienten am Ende eines schweren Verlaufes auf. Bei den meisten Menschen hat die Covid-19 Erkrankung keinen lebensbedrohlichen Verlauf. Allerdings wurde beobachtet, dass Covid-19 auch bei einem milden Krankheitsverlauf bleibende Lungenschäden verursachen kann (siehe u.a. Steffen Arora, "Tauchmediziner warnt vor gefährlichen Folgen einer Covid-19-Infektion", "DER STANDARD", 17. April 2020). Somit wäre ein Medikament, welches aufgrund seiner guten Verträglichkeit auch schon prophylaktisch und/oder bei milden Verläufen bzw. am Anfangsstadium einer Infektion verabreicht werden kann, von großem medizinischem Vorteil.

Ergänzend wird versucht, das Risiko für eine Ansteckung mit SARS-CoV-2 durch verschiedene Maßnahmen zu verringern, z.B. gründliche Händehygiene, Abstandhalten zu anderen Personen, das Tragen von einfachen Gesichtsmasken oder Flächenreinigung mit tensidhaltigen oder alkoholhaltigen Desinfektionsmitteln. Nachteilig an diesen Verfahren ist, dass die Gesichtsschutzmasken aufgrund der erhöhten Nachfrage in vielen Ländern nicht mehr in den Mengen verfügbar sind, in welchen sie eigentlich benötigt werden. Neben der Knappheit der Masken bewirken oft auch die Arbeitsweise in der Klinik oder mangelnde Kenntnis der Gefahren des wiederholten oder dauerhaften Tragens der gleichen Maske, dass Gesichtsmasken länger getragen werden, als dies unter hygienischen Gesichtspunkten sinnvoll ist. Durch falsche Handhabung von Masken kann sich die Gefahr einer Covid-19 Erkrankung sogar erhöhen. Bei der großflächigen Anwendung alkoholhaltiger Desinfektionsmittel erhöht sich zudem die Brandgefahr, und sowohl alkoholhaltige als auch tensidhaltige Lösungen haben die Eigenschaft, bei häufigem Auftragen auf die Haut die Haut auszutrocknen. Die Einatmung von derartigen Desinfektionsmitteln kann die Lunge schädigen.

### Technisches Problem und grundlegende Lösungen

Demgegenüber hat sich die Erfindung die Aufgabe gestellt, eine verbesserte Zusammensetzung und entsprechendes Verfahren zur vorbeugenden oder akuten Behandlung einer Covid-19 Erkrankung bereitzustellen, welche die vorgenannten Nachteile der im Stand der Technik bekannten Verfahren nicht oder nur in einem geringeren Maße aufweist.

Die der Erfindung zugrunde liegenden Aufgaben werden jeweils mit den Merkmalen der unabhängigen Patentansprüche gelöst. Ausführungsformen der Erfindung sind in den abhängigen Ansprüchen angegeben. Die im Folgenden aufgeführten Ausführungsformen sind frei miteinander kombinierbar, sofern sie sich nicht gegenseitig ausschließen.

In einem Aspekt betrifft die Erfindung ein Verfahren zur akuten oder vorbeugenden Behandlung von Covid-19. Das Verfahren beinhaltet:
- a) Erzeugung eines Aerosols an einem Ort, wo das Aerosol von zumindest einem Menschen inhaliert werden kann, wobei das Aerosol aus flüssigen oder festen Schwebeteilchen besteht, die zumindest ein Isochinolinalkaloid beinhalten; und/oder
- b) Behandlung eines Objektes mit einer wässrigen Lösung, die zumindest ein Isochinolinalkaloid beinhaltet, oder einem Aerosol, das zumindest ein Isochinolinalkaloid beinhaltet, wobei das Objekt ein Gerät, Kleidungsstück oder Gegenstand ist, das für den Kontakt mit der menschlichen Haut bestimmt ist.

Dies kann vorteilhaft sein, da Isochinolinalkaloide eine starke interkalierende Wirkung haben. Isochinolinalkaloide hemmen nicht nur die DNA-Replikation, sondern auch die Reverse Transkription und Replikation des einzelsträngigen RNA Virus SARS-CoV-2. Nach ihrer Absorption in eine Zelle können Isochinolinalkaloide die Replikation und Transkription eines Virus innerhalb seiner Wirtszelle beeinflussen bzw. hemmen.

Isochinolinalkaloide sind in der Natur vorkommende Substanzen, die auch in großen Mengen günstig synthetisch hergestellt oder aus Pflanzen extrahiert werden können. Sie sind biologisch abbaubar und erhöhen im Gegensatz zu alkoholischen Desinfektionsmitteln nicht die Brandgefahr.

Außerdem wurde beobachtet, dass Isochinolinalkaloide vergleichsweise schlecht von Epithelzellen (in Darm und Lunge) absorbiert werden. Dies kann im Kontext der Verwendung als Bestandteil eines Aerosols besonders vorteilhaft sein, denn der Wirkstoff erreicht zielgenau im Wesentlichen nur diejenigen Zellen, die er auch erreichen soll, nämlich die Zellen des Lungenepithelgewebes, wo sich auch die SARS-CoV-2 Viren befinden. Die interkalierenden Eigenschaften der Isochinolinalkaloide haben zwar den erwünschten virostatischen Effekt auf SARS-CoV-2 Viren, es kann derzeit aber nicht ausgeschlossen werden, dass dieser Effekt in menschlichen Zellen zu DNA-Doppelstrangbrüchen führt, was zu Zelltod und Krebs führen kann. Der Umstand, dass inhalierte Isochinolinalkaloide eine vorwiegend lokale Wirkung haben und potentielle Nebenwirkungen wie z.B. DNA-Doppelstrangbrüche ebenfalls lokal begrenzt wären, macht diese Substanzgruppe besonders geeignet für die prophylaktische Anwendung und für die Akutbehandlung von Covid-19 Patienten mit milden Symptomen, bei welchen ein Medikament mit starken Nebenwirkungen als inakzeptabel angesehen wird.

Während die Inhalation alkoholhaltiger oder tensidhaltiger Lösungsmittel das Lungengewebe reizt und Entzündungen hervorrufen können, die den ohnehin durch die Virusinfektion belasteten Patienten noch mehr belasten, wirken Isochinolinalkaloide entzündungshemmend. Dies kann zum einen bei der Vermeidung oder Behandlung des gefürchteten Zytokinsturms bei manchen schweren Verläufen der Covid-19 Erkrankung helfen.

Die Verwendung von isochinolinalkaloidhaltigen Aerosolen zur Bekämpfung von SARS-CoV-2 Viren ist aber auch eine sehr vorteilhafte Eigenschaft bei der Verabreichung des Aerosols als Raumspray bzw. zur großflächigen Desinfektion von Mobiliar, Straßen, Gebäudefassaden, und/oder den Innen- oder Außenwänden von Fahrzeugen, Gebäuden oder Messehallen. Sogar wenn man Desinfektionsmittel in flüssiger Form, also z.B. als wässrige Lösung mit einer Spritzflasche oder Spritzpumpe großflächig aufträgt, entstehen unvermeidbar Aerosole. Dies liegt daran, dass die Flüssigkeit mit einem gewissen Druck das Spritzgerät verlassen muss, um Objekte in einem größeren Umkreis, also z.B. im Umkreis von einem oder mehreren Metern, zu erreichen. Wenn der Strahl dabei mit hohem Druck auf ein Objekt trifft, werden unweigerlich Aerosole gebildet. Solche Aerosole sind für die Lungen von Personen, die die großflächige Desinfektion durchführen oder unmittelbar nach der Desinfektion in die Nähe der desinfizierten Objekte geraten, schädlich, wenn das verwendete Desinfektionsmittel Alkohol oder Tenside in nennenswerten Mengen enthalten. Isochinolinalkaloidhaltige Desinfektionsmittel dagegen haben dagegen einen positiven, nämlich viruziden und entzündungshemmenden Effekt auf die Lungen der Personen, die die Desinfektion durchführen oder unmittelbar nach der Desinfektion in die Nähe des desinfizierten Objektes kommen.

In einem weiteren vorteilhaften Aspekt wirken Isochinolinalkaloide nicht nur antiviral, sondern auch entzündungshemmend. Das isochinolinalkaloidhaltige Aerosol kann also sowohl die SARS-CoV-2-Infektion selbst als auch deren Begleiterscheinungen, insbesondere Entzündungen, bekämpfen. Dies kann insbesondere bei einem schweren Verlauf mit Zytokinsturm vorteilhaft sein und die Überlebenschancen der Patienten erhöhen. Die breite antimikrobielle Wirkung der Isochinolinalkaloide kann zudem dazu beitragen, bakterielle Sekundärinfektionen wirksam zu bekämpfen.

### Verfahren nach 1a)

Gemäß Ausführungsformen nach Option 1a) wird das Aerosol von einem medizinischen Zerstäuber, insbesondere einem portablen Zerstäuber, erzeugt.

Bei dem Zerstäuber kann es sich z.B. um eine Nasensprayflasche oder eine Rachensprayflasche mit Zerstäuberaufsatz handeln. Der Zerstäuberaufsatz kann z.B. in den Deckel der Flasche integriert sein. Bei dem Zerstäuberaufsatz kann es sich z.B. um einen Pumpzerstäuber handeln.

Bei dem medizinischen Zerstäuber kann es sich auch um ein Inhalationsgerät für die Anwendung zu Hause oder im Krankenhaus, das mit verschiedenen Flüssigkeiten befüllt werden kann, handeln. Derartige Inhalationsgeräte gibt es z.B. in der Form von elektrischen Inhalationsgeräten. Beispielsweise gibt es vom Hersteller PARI eine Reihe von Inhalationsgeräten, die mit Salzlösungen und verschiedenen Medikamenten befüllt werden können und die die Salzlösung bzw. sonstige Lösungen mit einem Medikament in die Atemwege eines Menschen transportieren können.

Bei dem portablen Zerstäuber kann es sich z.B. um eine Sprühdose oder einen Verstäubungsapparat handeln. Vorzugsweise hat der Apparat Dimensionen, die eine Mitführung in einer Handtasche oder Hosentasche erlauben. Beispielsweise ist die längste Seite des Apparats kürzer 20 cm, vorzugsweise kürzer als 15 cm. Dies ermöglicht einen flexiblen Einsatz zur Verabreichung des isochinolinalkaloidhaltigen Aerosols über die Atemwege an Menschen. Bei dem portablen Apparat kann es sich z.B. um eine Nasenspraydose handeln, also um eine Sprühdose mit einem Aufsatz zur Einführung in die Nasenöffnung von Menschen.

Vorzugsweise wird das Aerosol in hinreichender räumlicher Nähe zum Gesicht eines Menschen erzeugt, sodass sichergestellt ist, dass bei gegebener Isochinolinalkaloidkonzentration in dem Aerosol der Mensch eine physiologisch wirksame Menge des Isochinolinalkaloids über die Atemwege aufnimmt. Die notwendige räumliche Nähe hängt von Faktoren wie z.B. der Isochinolinalkaloidkonzentration, des Vernebelungs- bzw. Verstäubungsmechanismus und dem Anwendungskontext (Nasenspray, elektrisches Inhalationsgerät) ab. Eine hinreichende räumliche Nähe bedeutet nach Ausführungsformen der Erfindung, dass der portable Apparat einen Maximalabstand von 1 m, vorzugsweise 50 cm, weiterhin vorzugsweise 20 cm von der Nase des Menschen hat, wenn das Aerosol erzeugt wird.

Bei manchen Ausführungsformen handelt es sich bei dem portablen Apparat um einen Vernebelungs- oder Verstäubungsapparat mit einem Aufsatz, der in die Nasenöffnung oder den Mund des Menschen einzuführen ist ("Nasenspray", "Rachenspray"). Derartige Sprays erlauben eine gezielte Applikation auch hoher Dosen an bestimmte Personen. Dies kann zur prophylaktischen oder therapeutischen Behandlung dienen.

Diverse Mechanismen, die eine Vernebelung von Flüssigkeiten oder von festen Aerosolen durch portable Apparate ermöglichen, sind im Stand der Technik bekannt (siehe z.B. DE202005009619U1, DE202006015002U1, DE000003531568A1, DE102010042360A1).

Gemäß Ausführungsformen bei welchen das Aerosol zur Inhalation durch eine oder mehrere Personen bestimmt ist, ist die Konzentration des zumindest einen Isochinolinalkaloids in dem Aerosol und die Menge des vernebelten oder versprühten Aerosols so eingestellt sind, dass mindestens 0,1 mg, vorzugsweise mindestens 0,5 mg, vorzugsweise mindestens 1 mg des zumindest einen Isochinolinalkaloids pro Tag und Person inhaliert werden. Die Dosen können aber auch deutlich höher liegen von bis zu 1 bis 2 Gramm pro Person und Tag.

Gemäß Ausführungsformen werden zwischen 0,1 mg und 1000 mg, insbesondere zwischen 0,1 mg und 100 mg des zumindest einen Isochinolinalkaloids pro Tag und Person, insbesondere für prophylaktische Zwecke, vernebelt bzw. verstäubt.

Gemäß Ausführungsformen werden zwischen 0,8 mg und 2000 mg, insbesondere zwischen 0,2 mg und 2 g, z.B. zwischen 2 mg und 1 g, des zumindest einen Isochinolinalkaloids pro Tag und Person, insbesondere für eine Akutbehandlung (therapeutischen Zweck), vernebelt bzw. verstäubt.

Es wird bei den hier beschriebenen Ausführungsformen von einem Durchschnittsgewicht der Person von ca. 80 kg ausgegangen. Die Dosis kann für leichtere oder schwerere Personen entsprechend angepasst werden.

Nach Ausführungsformen wird die gesamte tägliche Menge über den portablen Apparat diskontinuierlich abgegeben, z.B. mittels 1-20, vorzugsweise 1-5 Vernebelungs- bzw. Verstäubungsvorgängen. Diese können manuell, z.B. durch Betätigung eines mechanischen Hebels oder eines sonstigen Elements des Apparats, initiiert werden.

Die Verstäubung oder Vernebelung kann aber auch kontinuierlich über den Tag durchgeführt werden, z.B. durch Zugabe einer isochinolinalkaloidhaltigen Zusammensetzung in eine kontinuierlich betriebene Vernebelungs- oder Verstäubungsanlage. Je nach Schwere der Erkrankung oder ob es sich um eine prophylaktische Maßnahme handelt, kann die vernebelte oder verstäubte Menge der Isochinolinalkaloidhaltigen Substanz variiert werden.

Beispielsweise kann ein isochinolinalkaloidhaltiges Nasen- oder Rachenspray bereitgestellt werden, das eine Dosierempfehlung enthält, wie oft am Tag und wann ein Atemzug des Aerosols eingeatmet werden soll. Die Konzentration der Isochinolinalkaloide in der wässrigen Lösung und der Zerstäubungsmechanismus und die Dosierempfehlung sind dabei so aufeinander abgestimmt, dass eine Person bei bestimmungsgemäßer Verwendung des Nasen- oder Rachensprays die oben bezeichnete Tagesdosis an Isochinolinalkaloiden einatmet.

Eine gemäß OECD-Richtlinien angelegte Studie an Ratten hat gezeigt, dass man kurz- oder langfristig bis zu 2000 mg einer aus Sanguinarin und Chelerythrin bestehenden Isochinolinalkaloid-Zusammensetzung je kg Lebendmasse verabreichen könnte, ohne dass ein relevanter Parameter der klinischen Toxikologie reagiert hätte, was auf Mensch oder Tier übertragen Tagesdosierungen an Isochinolinalkaloid - Alkaloiden von 150 g je Mensch und Tag erlauben würde. Somit ist die erfindungsgemäße Zusammensetzung somit im Hinblick auf die Nebenwirkungen unbedenklich.

### Verfahren nach 1b)

Gemäß Ausführungsformen nach Option 1b) ist das behandelte Objekt ausgewählt ist aus einer Gruppe umfassend:
- Medizinische Gesichtsmasken, insbesondere FFP2 und FFP3 Masken;
- Operations-Masken und Alltagsgesichtsmasken mit und ohne Filtereinsatz;
- Sitze, Haltestangen, Haltegriffe und sonstige Vorrichtungen innerhalb von Land-, Luft- und Wasserfahrzeugen, insbesondere von Bussen, Zügen, Flugzeugen und Passagierschiffen;
- Mobiliar von Gebäuden, insbesondere von Restaurants, Schulen, Kindergärten, Heimen, Firmen und Behörden sowie Hallen und Säle für Großveranstaltungen;
- Betttücher oder Handtücher;
- Türgriffe;
- Medizinische Verbände;
- Medizinische Einwegartikel, insbesondere Schutzkleidung.

Unter einer "Alltagsmaske" wird hier ein geschneidertes Stück Stoff, das über Kinn, Mund und Nase getragen wird, verstanden. Sie besteht meistens aus Baumwollstoff, der in Falten gelegt vernäht oder an die Gesichtsform angepasst geschneidert ist, und wird mit Gummibändern an den Ohren oder mit Haltebändern am Hinterkopf fixiert. Weitere Bezeichnungen sind Community-Maske, Mund-Nasen-Bedeckung (MNB), Behelfs-Mund-Nasen-Maske und Behelfsmaske.

Unter "medizinischen Gesichtsmasken" werden hier jegliche Masken verstanden, die im medizinischen Bereich verwendet werden und dem Selbst- und/oder Fremdschutz vor Infektionen verschiedenster Art dienen, insbesondere Mund-Nasen-Schutz (MNS) Masken und Atemschutzmasken wie FFP1, FFP2- und FFP3-Masken.

Gemäß manchen Ausführungsformen umfasst die Behandlung aus einem Tränken des Objekts mit der wässrigen Lösung, eine Immersion des Objekts in der wässrigen Lösung oder ein Abwischen des Objekts mit der wässrigen Lösung.

Beispielsweise werden große Oberflächen wie z.B. Tischplatten, Stühle, das Mobiliar von Gebäuden und Fahrzeugen, vorzugsweise großflächig mit einer isochinolinalkaloidhaltigen Lösung abgewischt oder mit einem entsprechenden Aerosol besprüht. Bei manchen porösen, saugfähigen oder mechanisch instabilen Oberflächen wie z.B. Gesichtsmasken, Schutzkleidung, Betttücher, Handtücher, Verbände etc. kann es dagegen sinnvoll sein, diese in eine isochinolinalkaloidhaltigen Lösung einzutauchen oder sie mit einem isochinolinalkaloidhaltigen Aerosol zu behandeln. Das Eintauchen hat den Vorteil, dass der Wirkstoff tiefer in das Gewebe eindringt und so tendenziell eine größere Wirkstoffmenge auf bzw. in das Objekt auf- oder eingebracht werden kann. Allerdings ist die Trocknungszeit größer als bei einer oberflächlichen Behandlung mit einem Aerosol.

Die Behandlung der oben genannten Objekte, insbesondere von Gesichtsmasken, Schutzkleidung und medizinischen Einmalartikeln kann den Vorteil haben, dass die Gefahr einer Schmierinfektion mit SARS-CoV-2 deutlich reduziert werden kann. Sogar falls der Träger einer Gesichtsmaske beim An- oder Ablegen der Maske die innere oder äußere Oberfläche der Maske mit dem Erreger kontaminieren sollte, wird dieser binnen kurzer Zeit unschädlich gemacht.

Nach Ausführungsformen wird das Aerosol aus Wasser und einer Isochinolinalkaloid-haltigen Zusammensetzung, z.B. einem flüssigen Konzentrat, erst am Ort der Erzeugung des Aerosols derart hergestellt, dass das Isochinolinalkaloidkonzentrat über eine automatische Dosieranlage dem Vernebelungswasser beigemischt wird. Durch dieses Verfahren kann auch jederzeit die Anwendungskonzentration des isochinolinalkaloidhaltigen Aerosols wechselnden Bedingungen wie Umgebungstemperatur, Luftfeuchtigkeit, Anzahl der Personen pro Bodenfläche etc. angepasst werden, sodass die Zudosierung des isochinolinalkaloidhaltigen Konzentrats zum Aerosol entsprechend erhöht oder erniedrigt werden kann.

Nach Ausführungsformen der Erfindung gemäß Option 1b) sind bei der Aerosolerzeugung die Konzentration des zumindest einen Isochinolinalkaloids in dem Aerosol und die Menge des vernebelten oder versprühten Aerosols so eingestellt, dass mindestens 0,1 mg des zumindest einen Isochinolinalkaloids pro Quadratmeter Bodenfläche und pro Tag oder pro Quadratmeter Objekt und Tag verstäubt werden.

Es wurde beobachtet, dass diese vergleichsweise geringe Menge von Isochinolinalkaloiden bereits ausreicht, um einen deutlichen Effekt auf die Menge der pro Flächeneinheit noch nachweisbaren SARS-CoV-2 Viren zu haben. Es ist aber auch möglich, z.B. bei besonders sensiblen Bereichen (Aufzug) oder Objekten (Gesichtsmasken), deutlich höhere Konzentrationen Isochinolinalkaloid in der Zusammensetzung zu verwenden, z.B. mindestens 1 mg oder mindestens 100 mg Isochinolinalkaloid pro behandelter Quadratmeter Objektoberfläche.

Nach Ausführungsformen der Erfindung sind die Konzentration des zumindest einen Isochinolinalkaloids in dem Aerosol und die Menge des vernebelten oder versprühten Aerosols so eingestellt, dass weniger als 2 g, z.B. weniger als 1 mg des zumindest einen Isochinolinalkaloids pro Quadratmeter Bodenfläche oder Objektfläche und pro Tag verstäubt werden.

Gerade bei einer großflächigen Anwendung ist eine geringe Konzentration im Hinblick auf die Kosten ein wichtiger und vorteilhafter Aspekt.

Falls z.B. 0,1 mg Isochinolinalkaloid pro Quadratmeter Boden oder Objektoberfläche und Tag (oder pro Behandlung) vernebelt oder verstäubt werden sollen, kann dies z.B.: gemäß folgender Optionen erreicht werden:
- Falls die vernebelte oder verstäubte Substanz z.B. 1 % Isochinolinalkaloid enthält, werden 10 mg dieser Substanz je Quadratmeter und Tag vernebelt bzw. verstäubt.
- Falls die vernebelte oder verstäubte Substanz z.B. 0,01 % Isochinolinalkaloid enthält, werden 1 g dieser Substanz je Quadratmeter und Tag vernebelt bzw. verstäubt.

Nach Ausführungsformen wird das Objekt, z.B. bei der Herstellung oder zum Zwecke der Wiederaufbereitung zum mehrfachen Gebrauch, in eine wässrige Isochinolinalkaloidlösung getaucht oder damit einseitig oder mehrseitig besprüht. Hier können ebenfalls Isochinolinalkaloidkonzentrationen von 0,1 mg bis z.B. 20 g pro Liter der wässrigen Lösung verwendet werden. Hier kann die Isochinolinakaloidkonzentration also recht hoch sein. Da das behandelte Objekt weder inhaliert noch verzehrt wird, ist die Applikation auch von sehr hochkonzentrierten Isochinolinalkaloidkonzentrationen gesundheitlich unbedenklich.

### Verfahren nach 1a und/oder 1b

Nach Ausführungsformen der Erfindung wird das Aerosol von einer Vorrichtung zur Belüftung von Gebäuden, insbesondere von einer Klimaanlage oder Luftfilteranlage, erzeugt.

Dies kann vorteilhaft sein, da hier der gleiche Vorgang sowohl zu einer Reduktion der Viruslast auf Objektoberflächen (z.B. Tischplatten, Tastaturen, Bildschirme, Türen, Stühle etc.) als auch zur prophylaktischen oder therapeutischen Behandlung von Personen dienen kann. Beispielsweise kann eine kontinuierliche Vernebelung geringer Isochinolinalkaloidmengen in einem Bürogebäude oder Großraumbüro oder in besonders infektionsträchtigen Bereichen wie Aufzug oder Treppenhaus dafür sorgen, dass die Ansteckungsgefahr durch Schmierinfektion minimiert wird und Personen, die sich möglicherweise bereits infiziert haben ohne es zu merken in ihrem Heilungsprozess unterstützt werden. Es ist auch möglich, dass die Vorrichtung zur Belüftung von Gebäuden das Aerosol nur während bestimmter Zeiträume erzeugt und/oder verbreitet. Beispielsweise könnte dieser Zeitraum frühmorgens sein, bevor die ersten Mitarbeiter das Gebäude betreten, oder am Abend, wenn die Mitarbeiter das Gebäude bereits verlassen haben. Falls sich auf Tischplatten, Tastaturen oder sonstigen Objekten mit SARS-CoV-2 kontaminiertes Material befinden sollte, wird dieses in dem Zeitraum zwischen zwei Arbeitstagen durch das isochinolinalkaloidhaltige Aerosol vollständig oder weitgehend unschädlich gemacht.

Gemäß einer Ausführungsform wird die Aerosolerzeugung so durchgeführt, dass pro Tag ein Aerosol mit 0,1 mg Isochinolinalkaloid pro Quadratmeter Boden erzeugt wird. Geht man gemäß einer groben Abschätzung davon aus, dass eine Person ca. einen Quadratmeter belegt und die Person etwa die Hälfte davon einatmet, werden der Person auf diese Weise 0,05 mg Isochinolinalkaloid täglich verabreicht. Dies kann durchaus eine vor SARS-CoV-2 schützende Wirkung auf die Person haben, auch wenn der Hauptzweck der Vernebelung z.B. eine Desinfektion von Objektoberflächen war.

Nach einer alternativen Ausführungsform wird das Aerosol von einer Aerosolerzeugungseinheit erzeugt, welche für die Installation auf einem Fahrzeug oder für den Transport durch eine Person bestimmt ist. Beispielsweise kann es sich bei der Aerosolerzeugungseinheit um eine Vorrichtung handeln, die eine Person auf dem Rücken tragen kann wie einen Rucksack, oder um einen Aufsatz, der sich auf einem PKW oder LKW befestigen lässt. Die Zerstäubungseinheit beinhaltet:
- einen Tank mit einer Zusammensetzung mit dem zumindest einen Isochinolinalkaloid; Die Zusammensetzung kann z.B. eine wässrige Lösung oder ein Pulver sein; In ersterem Fall wird die Aerosolerzeugungseinheit auch als Vernebelungseinheit bezeichnet, im letztgenannten Fall auch als Zerstäubungseinheit;
- eine Düse, wobei die Düse mit dem Tank leitend verbunden ist und dazu ausgebildet ist, die Zusammensetzung in ein Aerosol umzuwandeln (z.B. zu vernebeln oder zu zerstäuben); die Düse kann auch mehrere Düsen umfassen; die Düse ist vorzugsweise verschwenkbar gelagert oder so ausgebildet, dass sie von Hand zu mehreren Richtungen hin ausgerichtet werden kann; und
- eine Druckerzeugungseinheit, die dazu ausgebildet ist, die Zusammensetzung oder das Aerosol mit einem Druck zu beaufschlagen, der hinreichend ist, um das Aerosol in einem Radius von mindestens 0,5 m, vorzugsweise mindestens 1 m um die Düse herum, in mindestens eine Richtung zu verbreiten.

Dies kann vorteilhaft sein, da große Flächen, z.B. Straßenzüge, Turnhallen, Messegelände, Sportstadien und dergleichen auf effiziente Weise desinfiziert werden können.

Nach Ausführungsformen der Erfindung handelt es sich bei dem zumindest einen Isochinolinalkaloid um Sanguinarin, Chelerythrin, Chelirubin, Chelidonin, Chelilutin, Cholerythrin, Berberin, Protoberberin, Protopin, α- Allocryptopin, β-Allocryptopin, Macarpin, Cularin oder eine Kombination von zwei oder mehr der vorgenannten Isochinolinalkaloide.

Nach Ausführungsformen der Erfindung bestehen mindestens 10%, vorzugsweise mindestens 30% des zumindest einen Isochinolinalkaloids aus Sanguinarin, Berberin und/oder Chelerythrin. Nach Ausführungsformen der Erfindung bestehen mindestens 90% des zumindest einen Isochinolinalkaloids aus Sanguinarin, Berberin und/oder Chelerythrin.

Die Verwendung dieser Isochinolinalkaloide kann vorteilhaft sein, da beobachtet wurde, dass diese Alkaloide eine starke antivirale Wirkung bei vergleichsweise geringen Nebenwirkungen haben. Außerdem sind diese Alkaloide günstig in großen Mengen verfügbar. Sanguinarin und teilweise auch Chelerythrin können aus Extrakten von Sanguinaria canadensis, Macleaya cordata, Bocconia frutescens, Eschscholzia californica und Chelidonium majus gewonnen werden. Berberin kann aus vielen Arten der Berberidaceae und Papaveraceae extrahiert werden.

Nach Ausführungsformen beinhaltet die Zusammensetzung das zumindest eine Isochinolinalkaloid als Bestandteil eines Extrakts, der aus Pflanzenmaterial einer Pflanze gewonnen wurde. Die Pflanze kann z.B. sein: die im vorigen Paragraph genannten, sowie Macleaya microcarpa; Argemone mexicana; Mitglieder der Berberis-Pflanzengattung, insbesondere Berberis vulgaris; Hydrastis canadensis; Mitglieder der Coptis-Pflanzengattung; insbesondere Coptis chinensis; Mitglieder der Argemone-Pflanzengattung; insbesondere Argemone mexicana; und Philodendron.

Die Extraktion kann z.B. mittels Ethanolextraktion erfolgen. Es folgt ein Schritt der Kristallisation des zumindest einen extrahierten Isochinolinalkaloids mittels einer Säure zu einem Isochinolinalkaloid-Salz. Die Säure kann z.B. Salzsäure, Schwefelsäure oder Tanninsäure sein, entsprechend können die resultierenden Salze ein Alkaloid -chlorid, -sulfat oder -tannat sein, wobei das Alkaloid insbesondere aus Chelerythrin und Sanguinarin bestehen kann. Das Salz wird nun als isochinolinalkaloidhaltiges Extrakt verwendet um die Zusammensetzung nach einer der beschriebenen Ausführungsformen bereitzustellen.

Nach Ausführungsformen umfasst die Bereitstellung des zumindest einen Isochinolinalkaloids:
- Extraktion des zumindest einen Isochinolinalkaloids aus Pflanzenmaterial von Pflanzen einer der oben genannten Pflanzengruppen;
- Kristallisation des zumindest einen extrahierten Isochinolinalkaloids mittels einer Säure zu einem Isochinolinalkaloid-Salz; und
- Verwenden des Isochinolinalkaloid-Salzes um das zumindest eine Isochinolinalkaloid bereitzustellen.

Nach Ausführungsformen der Erfindung besteht das Aerosol aus flüssigen Schwebeteilchen, die von einer Vernebelungsanlage durch Vernebelung einer wässrigen Isochinolinalkaloidlösung erzeugt werden. Das Aerosol kann z.B. mit einer Zerstäuberflasche, einer Hochdruckvernebelungsanlage und/oder Luftkühlanlage mit Vernebelungsfunktion erzeugt werden.

Nach Ausführungsformen ist die Vorrichtung zur Vernebelung eine auf dem Verdunstungsprinzip beruhende Luftkühlanlage für Innen- und Außenbereiche von Gebäuden. Insbesondere kann es sich bei der Luftkühlanlage um eine Hochdruckvernebelungs-Luftkühlanlage handeln. Beispielhaft für solche Hochdruckvernebelungssysteme seien hier AFT-Nebelsysteme der Firma AFT GmbH & Co. KG genannt. Hochdruckvernebelungssysteme erzeugen durch Versprühen von Wasser aus Düsen unter hohem Druck (oft 70bar und mehr) einen feinen Nebel, der den Vorteil hat, dass die Schwebeteilchen lange in der Luft verbleiben bevor sie sich absetzen.

Dies kann vorteilhaft sein, da solche Luftkühlanlagen in manchen Gebäuden und Hallen bereits vorinstalliert und vorhanden sind. Es entfällt also die Anschaffung oder Installation zusätzlicher Anlagen um das Aerosol zu generieren.

Nach einer alternativen Ausführungsform ist das das Aerosol ein Feststoffaerosol, das insbesondere durch Verstäubung des zumindest einen Isochinolinalkaloids oder dessen Salzes erzeugt wird. Beispielsweise kann das Aerosol dadurch erzeugt werden, dass eine isochinolinalkaloidhaltige Substanz oder Zusammensetzung zu einem Pulver mit einem Korndurchmesser der Mehrzahl der Pulverkörner von zwischen 0,5 nm und mehreren 10 µm, weiterhin vorzugsweise zwischen 0,5-10 µm, zermahlen wird. Das Pulver kann direkt verstäubt werden oder in Wasser in Lösung gebracht und das Aerosol als Tröpfchenaerosol erzeugt werden.

Der Durchmesser der Mehrzahl der Schwebeteilchen liegt (sowohl beim FeststoffAerosol als auch beim Tröpfchen-Aerosol) vorzugsweise zwischen 0,5 nm und mehreren 10 µm, weiterhin vorzugsweise zwischen 0,5-10 µm. Dies kann vorteilhaft sein, da in diesem Größenbereich eine sehr gute Lungengängigkeit erzielt werden kann.

In einem weiteren Aspekt betrifft die Erfindung eine Zusammensetzung zur akuten oder vorbeugenden Behandlung von Covid-19, wobei die Zusammensetzung zumindest ein Isochinolinalkaloid beinhaltet.

Nach Ausführungsformen ist die Zusammensetzung eine wässrige Lösung, die das zumindest eine Isochinolinalkaloid beinhaltet.

Nach einer anderen Ausführungsform ist die Zusammensetzung ein Pulver, das das zumindest eine Isochinolinalkaloid beinhaltet.

Nach einer anderen Ausführungsform ist die Zusammensetzung ein Aerosol aus Pulverpartikeln oder Flüssigkeitströpfchen, wobei das Aerosol zumindest ein Isochinolinalkaloid beinhaltet.

In einem weiteren Aspekt betrifft die Erfindung ein Objekt. Das Objekt ist ein Gerät, ein Kleidungsstück oder ein Gegenstand, das bzw. der für den Kontakt mit der menschlichen Haut bestimmt ist. Das Objekt beinhaltet oder trägt zur Verhinderung einer Ansteckung mit Covid-19 zumindest ein Isochinolinalkaloid.

Nach Ausführungsformen ist das Objekt ausgewählt aus einer Gruppe umfassend:
- Medizinische Gesichtsschutzmasken, insbesondere FFP2 und FFP3 Masken;
- Operations-Masken und Alltags-Gesichtsmasken mit und ohne Filtereinsatz;
- Betttücher oder Handtücher;
- Medizinische Verbände;
- Medizinische Einwegartikel, insbesondere Schutzkleidung.

Dies kann vorteilhaft sein, da einer Schmierinfektion oder Tröpfcheninfektion durch SARS-CoV-2 besser vorgebeugt werden kann: falls ein Objekt mit dem Virus kontaminiert wird, wird das Virus mit hoher Wahrscheinlichkeit durch das Alkaloid abgetötet, bevor sich der Nutzer oder Träger des Objekts an der Kontamination infizieren kann.

In einem weiteren Aspekt betrifft die Erfindung ein Nasenspray, Rachenspray oder Raumspray zur Verhinderung einer Ansteckung mit oder zur Behandlung von Covid-19, beinhaltend:
- in portables Behältnis mit einer Zusammensetzung mit zumindest einem Isochinolinalkaloid, und
- einer Einheit zur Erzeugung eines Aerosols aus der Zusammensetzung.

Die Zusammensetzung kann z.B. eine wässrige Isochinolinalkaloidlösung sein oder ein Pulver, das so fein gemahlen ist, dass es zur Erzeugung eines Festkörperaerosols geeignet ist, das lange genug in der Luft steht, um inhaliert zu werden.

In einem weiteren Aspekt betrifft die Erfindung eine Vorrichtung zur Erzeugung eines Aerosols im Außenbereich oder Innenbereich von Gebäuden. Insbesondere kann es sich um eine Vorrichtung zur Erzeugung eines Aerosols in einem Fahrstuhl handeln. Die Vorrichtung umfasst:
- ein Behältnis mit einer Zusammensetzung mit zumindest einem Isochinolinalkaloid, (z.B. eine wässrige Lösung mit ein oder mehreren Isochinolinalkaloiden, oder ein Pulver mit ein oder mehreren Isochinolinalkaloiden); und
- einer Einheit zur Erzeugung eines Aerosols aus der Zusammensetzung.

Die Zusammensetzung dient der vorbeugenden und/oder akuten Behandlung von Covid-19. Die Vorrichtung kann z.B. als Klimaanlage oder Luftfilteranlage ausgebildet sein und neben der Funktion der Aerosolbildung noch weitere Funktionen wahrnehmen. Es ist aber auch möglich, dass die einzige oder wesentliche Funktion der Vorrichtung die Erzeugung des Aerosols ist. Eine solche Vorrichtung wird oft als Vernebelungsanlage oder Verstäubungsanlage bezeichnet.

Unter "Vernebelung" wird im Folgenden die Erzeugung eines Aerosols aus flüssigen Schwebeteilchen verstanden. Unter "Verstäubung" wird im Folgenden die Erzeugung eines Aerosols aus festen Schwebeteilchen verstanden.

### Isochinolinalkaloidhaltige Zusammensetzung

Es können verschiedene Formulierungen der isochinolinalkaloidhaltigen Zusammensetzung verwendet werden. Beispielsweise kann die Zusammensetzung einen Isochinolinalkaloidgehalt von 0,1 % aufweisen, aber auch ein Isochinolinalkaloidgehalt von 40 % und mehr ist möglich. Die feste oder flüssige Substanz kann dann allein oder nach Mischung mit weiteren Substanzen (Füllstoffe im Falle des festen Aerosols, Leitungswasser im Fall des flüssigen Aerosols) in Aerosolform gebracht werden.

Vorzugsweise sind die Düsen einer Vorrichtung zur Vernebelung oder zur Verstäubung des isochinolinalkaloidhaltigen Aerosols an der Decke oder Wand eines Gebäudes, eines Raumes oder eines Fahrstuhls oder auf einem Fahrzeug befestigt. Die Vorrichtung kann dazu ausgebildet sein, das Aerosol innerhalb oder außerhalb des Gebäudes zu erzeugen. Die Düsen sind vorzugsweise so ausgerichtet, dass eine möglichst homogene Verteilung des Aerosols insbesondere in den Bereichen, in denen sich Menschen aufhalten, bewirkt wird.

### Aerosol aus festen Schwebeteilchen

Nach Ausführungsformen besteht das Aerosol aus festen Schwebeteilchen, die von einer Verstäubungsanlage durch Verstäubung des zumindest einen Isochinolinalkaloids oder dessen Salzes, das z.B. in Pulverform vorliegt, erzeugt wurde. Das Pulver kann z.B. aus dem Salz des Isochinolinalkaloids gewonnen werden, oder aus einer sonstigen isochinolinalkaloidhaltigen Zusammensetzung z.B. durch Anwenden eines Mahlprozesses. Das Pulver kann auch weitere Bestandteile, insbesondere Füllstoffe enthalten, die die Verstäubung und Dosierung erleichtern. Vorzugsweise handelt es bei den Füllstoffen um Stoffe, die über die Lunge als physiologische und unproblematische Stoffe aufgenommen werden können wie z. b. physiologisch verträgliche Bindemittel, diverse Vitamine, Extrakte aus weiteren Heilpflanzen wie Kamille, Efeu oder Eukalyptus die ihrerseits z. B. als Schleimlösend bekannt sind oder physiologische Zucker wie Traubenzucker (Dextrose). Vorzugsweise sind sämtliche Füllstoffe wasserlöslich und/oder fein vermahlen, sodass sie umgehend absorbiert werden können und Bakterien keinen Nährboden bieten.

Nach Ausführungsformen wird das Pulver dadurch erzeugt, dass eine isochinolinalkaloidhaltige Zusammensetzung auf einen Korndurchmesser der Mehrzahl der Pulverkörner von zwischen 0,5 nm und mehreren 10 µm, weiterhin vorzugsweise zwischen 0,5-10 µm, zermahlen wird.

Die pulverförmige Zusammensetzung beinhaltet nach Ausführungsformen nur ein oder mehrere Isochinolinalkaloide, optional gemischt mit Füllstoffen, und keine Säure.

Nach anderen Ausführungsformen enthält das Pulver zusätzlich zumindest eine Säure, z.B. eine pulverförmige Säure, z.B. eine pulverförmige hydrophile oder amphiphile anorganische oder organische Säure. Insbesondere kann es sich bei der Säure um eine Carbonsäure handeln, z.B. Äpfelsäure.

Ein derartiges Pulver kann den Vorteil haben, dass es als Grundlage zur Erzeugung einer wässrigen isochinolinalkaloidhaltigen Lösung dienen kann, die dann wiederum zur Bildung eines Tröpfchenaerosols dienen kann. Die Verwendung einer Säure hat den Vorteil, dass die Säure die Löslichkeit der Isochinolinalkaloide verbessert und eine Ausfällung der Alkaloide bei längerer Lagerzeit verhindert. Pulverkörner, die in die Lunge gelangen, erzeugen dort zumindest in ihrer unmittelbaren Umgebung im Zuge des Lösungsprozesses im wässrigen Milieu der Lunge eine saure wässrige Umgebung. Diese stabilisiert das Isochinolinalkaloid chemisch und wirkt zudem als Lösungsvermittler. Dadurch kann das eingeatmete Isochinolinalkaloid besser an die Zellen des Lungenepithels gelangen und dort wirken.

Nach manchen Ausführungsformen wird das Pulver in Wasser gelöst, sodass eine wässrige Isochinolinalkaloidlösung entsteht. Vorzugsweise beinhaltet diese Lösung mindestens eine Säure, z.B. eine Carbonsäure, z.B. Äpfelsäure, und hat einen pH-Wert von unter 6,8. Alternativ dazu kann das Pulver direkt verstäubt werden.

### Beispiele von auf Äpfelsäure beruhenden Zusammensetzungen

Nach einer Ausführungsform beinhaltet die Zusammensetzung zwischen 30 Gew.% ("Gewichtsprozent") und 96 Gew.% ("Gewichtsprozent") Äpfelsäure als die zumindest eine Säure und 4 Gew.% Pflanzenextrakt, wobei ca. 25% des Pflanzenextrakts aus Sanguinarin und/oder Chelerythrin bestehen; Der Anteil des zumindest einen Isochinolinalkaloids an der Zusammensetzung ist also 4 Gew.% ^{∗} 0,25 = 1 Gew.% an der gesamten Zusammensetzung. Das Verhältnis von Alkaloid zu Säure liegt bei 1:30 bis 1:96. Wird synthetisches Alkaloid verwendet, kann auch direkt 1 Gew% abgemessen und der Zusammensetzung beigemischt werden. Die Zusammensetzung kann mit weiteren Zusatzstoffen auf 100 Gew.% gebracht werden. Hierzu zählen Bindemittel, Geschmacksstoffe, insbesondere Süßstoffe und Zucker, Säurungsmittel, Salze, Aminosäuren und ähnliches.

Nach einer Ausführungsform beinhaltet die Zusammensetzung z.B.: 96 Gew.% ("Gewichtsprozent") Äpfelsäure als die zumindest eine Säure und 4 Gew.% Pflanzenextrakt, wobei 25% des Pflanzenextrakts aus Sanguinarin und Chelerythrin bestehen; Der Anteil des zumindest einen Isochinolinalkaloids an der Zusammensetzung ist also 4 Gew.% ^{∗} 0,25 = 1 Gew.%. Das Verhältnis von Alkaloid zu Säure liegt bei 1:96.

Nach einer weiteren Ausführungsform beinhaltet die Zusammensetzung z.B.: 94 Gew.% Äpfelsäure, 2 Gew. % Kaliumsorbat als Konservierungsmittel und 4 Gew.% Pflanzenextrakt, wobei 25 % des Pflanzenextrakts aus Sanguinarin und Chelerythrin bestehen. Das Verhältnis von Alkaloid zu Säure liegt bei 1:94.

Nach einer weiteren Ausführungsform beinhaltet die Zusammensetzung z.B.: 81 Gew.% Äpfelsäure, 2 Gew.% Kaliumsorbat als Konservierungsmittel, 1 Gew. % Natriumbicarbonat als Treibmittel in Brausetabletten, 11 Gew.% Mono-Natriumglutamat als Geschmacksverstärker, 1 Gew. % Natriumchlorid als Salz und 4 Gew.% Pflanzenextrakt, wobei 25% des Pflanzenextrakts aus Sanguinarin und Chelerythrin bestehen. Das Verhältnis von Alkaloid zu Säure liegt bei 1:81.

Nach einer weiteren Ausführungsform beinhaltet die Zusammensetzung z.B.: 77 Gew.% Äpfelsäure, 19 Gew% Sorbitol oder einem anderen Zuckeralkohol als der zumindest einen weiteren organischen Substanz und 4 Gew.% Pflanzenextrakt, wobei 25 % des Pflanzenextrakts aus Sanguinarin und Chelerythrin bestehen. Das Verhältnis von Alkaloid zu Säure liegt bei 1:77.

Nach einer weiteren Ausführungsform beinhaltet die Zusammensetzung z.B.: 60-70 Gew.% Äpfelsäure und 4 Gew.% Pflanzenextrakt, wobei 50 % des Pflanzenextrakts aus Sanguinarin und Chelerythrin bestehen. Weitere Substanzen, z.B. 19 Gew% Sorbitol oder ein anderer Zuckeralkohol, und/oder wenige Gew.% Salze, K-Sorbinsäure, Mononatriumglutamat können in der Zusammensetzung enthalten sein. Das Verhältnis von Alkaloid zu Säure liegt bei 1:30 bis 1:35.

Die oben genannten Mengenverhältnisse sind beispielhaft für die Alkaloide Sanguinarin/Chelerythrin und die Carbonsäure Äpfelsäure genannt. Im Falle der Verwendung anderer Isochinolinalkaloide, die eine andere physiologisch wirksame Konzentration aufweisen und/oder im Falle der Verwendung einer anderen Säure mit anderer Säurestärke kann das Gewichtsverhältnis von Alkaloid und Säure in der Zusammensetzung auch von den hier beispielhaft genannten Mischungen abweichen.

Die oben genannten Mengenverhältnisse können an die jeweilige medizinische Situation derart angepasst sein, dass z. b. der Anteil an isochinolinalkaloidhaltigen Pflanzenextrakt auch niedriger oder höher sein kann, je nachdem wie die spätere Anwendungsmenge es erfordert. Die Gewichtsprozente an Pflanzenextrakt in der zur Erzeugung des Aerosols und/oder zur Behandlung von Objekten verwendeten Zusammensetzung können somit von 0,1 % bis 10 % oder höher liegen wobei dann vorzugsweise der Anteil an Carbonsäure entsprechend erniedrigt oder erhöht wird um sicherzustellen, dass der pH-Wert der wässrigen Lösung unter 6,8 liegt. Falls das Pulver direkt zur Erzeugung eines festen Aerosols verwendet wird, ist der Anteil der Äpfelsäure vorzugsweise unter 10% um eine Reizung der Lunge zu vermeiden.

Nach Ausführungsformen wird die isochinolinalkaloidhaltige Zusammensetzung, die das Pulver ist oder aus welcher das Pulver durch einen Mahlprozess gewonnen wird, durch folgendes Verfahren gewonnen:
- Bereitstellung des zumindest einen Isochinolinalkaloids;
- Ermittlung einer Menge des zumindest einen Isochinolinalkaloids, die verdünnt in einer bestimmten Menge Wasser eine physiologisch wirksame wässrige Lösung des Isochinolinalkaloids ergibt;
- Bereitstellung zumindest einer Säure, z.B. Äpfelsäure;
- Ermittlung einer Menge der zumindest einen Säure, die bei Auflösung in der bestimmten Menge Wasser den pH-Wert dieser bestimmten Menge Wasser unter 6.8 bringt;
- Bereitstellung der Zusammensetzung, wobei die Zusammensetzung das zumindest eine Isochinolinalkaloid und die zumindest eine Säure enthält, wobei das Gewichtsverhältnis des zumindest einen Isochinolinalkaloids zu der zumindest einen Säure in der Zusammensetzung dem Gewichtsverhältnis der ermittelten Menge des zumindest einen Isochinolinalkaloids und der ermittelten Menge der zumindest einen Säure entspricht. Die Bereitstellung kann z.B. dadurch erfolgen, dass die zumindest eine Säure und das zumindest eine Isochinolinalkaloid in dem besagten Gewichtsverhältnis miteinander gemischt werden. Die sich ergebende Mischung, optional ergänzt um weitere Stoffe, ergibt die Zusammensetzung.

Vorzugsweise wird für die Ermittlung Wasser mit einem pH-Ausgangswert von 7.0 und/oder einem mittleren Härtegrad verwendet.

Somit wird eine Zusammensetzung erzeugt, die zumindest ein Isochinolinalkaloid und zumindest eine Säure beinhaltet. Das Gewichtsverhältnis des zumindest einen Isochinolinalkaloids zu der zumindest einen Säure und die Art der Säure ist so (also insbesondere unter Berücksichtigung der Säurestärke der Säure) gewählt, dass eine Auflösung der Zusammensetzung in einer bestimmten Menge Wasser (vorzugsweise mittlerer Härte und mit Ausgangs-pH-Wert von 7.0) den pH-Wert der bestimmten Menge Wasser auf unter 6.8 absenkt und die Konzentration des zumindest einen Isochinolinalkaloids hinreichend hoch ist um physiologisch wirksam zu sein, wenn eine bestimmte Menge des flüssigen Aerosols am Tag über die Lunge eingeatmet wird. Für manche Anwendungsszenarien ist eine Isochinolinalkaloidkonzentration von 0,01 mg Isochinolinalkaloid pro Liter Wasser bereits ausreichend um eine physiologische Wirkung zu zeigen.

Die oben genannte Zusammensetzung kann eine Stabilisierung von Isochinolinalkaloiden in wässrigem Milieu, wie dies z.B. in der Lunge vorherrscht, bewirken und für eine vollständige Auflösung des Isochinolinalkaloids in Wasser sorgen. Eine vollständige Auflösung der Zusammensetzung in Wasser kann vorteilhaft sein, da keine wasserunlöslichen Ablagerungen in der Lunge verbleiben. Außerdem kann die Stabilisierung von Isochinolinalkaloiden in wässrigem Milieu vorteilhaft, da die physiologische Wirksamkeit des Alkaloids im Behälter der Vernebelungsvorrichtung lange Zeit chemisch stabil ist. Außerdem beugt eine vollständige Lösung der Verstopfung der Düsen der Vernebelungsanlage oder der Düsen des portablen Apparats vor.

Nach Ausführungsformen wird die Menge der zumindest einen Säure so ermittelt, dass die ermittelte Menge der zumindest einen Säure bei Auflösung in der bestimmten Menge Wasser den pH-Wert dieser bestimmten Menge Wasser auf unter 6.5 bringt. Auch hier wird von einem pH-Wert des Wassers ohne die Zusammensetzung von 7 ausgegangen. Dies entspricht dem pH-Wert reinen Wassers. Dies kann vorteilhaft sein, da ein niedrigerer pH-Wert die Haltbarkeit und chemische Stabilität des Alkaloids weiter verbessert und sich auch bei der Verwendung von leicht alkalischem Wasser als Ausgangswasser zumeist ein pH-Wert von zumindest noch unter 6.8 einstellen lässt.

Nach Ausführungsformen wird die Menge der zumindest einen Säure so ermittelt, dass die ermittelte Menge der zumindest einen Säure bei Auflösung in der bestimmten Menge Wasser den pH-Wert dieser bestimmten Menge Wasser auf unter 6.5 bringt. Auch hier wird von einem pH-Wert des Wassers ohne die Zusammensetzung von 7 ausgegangen. Dies entspricht dem pH-Wert reinen Wassers. Vorzugsweise wird die Menge der Säure so ermittelt, dass sie den pH-Wert von Wasser mittlerer Wasserhärte auf unter 6.8 bzw. 6.5 bringt. Dies kann vorteilhaft sein, da ein niedrigerer pH-Wert die Haltbarkeit und chemische Stabilität des Alkaloids weiter verbessert und sich auch bei der Verwendung von leicht alkalischem Wasser und/oder höheren Härtegraden als Ausgangswasser zumeist ein pH-Wert von zumindest noch unter 6.8 einstellen lässt. Die Verwendung dieser Heuristiken (also eine Einstellung des Säure- zu Alkaloidverhältnisses unter Annahme eines pH-Werts von 7 und mittlerer Wasserhärte kann vorteilhaft sein, da die Wasserbeschaffenheit beim Endabnehmer einer als Konzentrat bereitgestellten Zusammensetzung oft nicht genau bekannt ist.

Beispielsweise liegt das Gewichtsverhältnis des zumindest einen Isochinolinalkaloids zu der zumindest einen Säure in der Zusammensetzung zwischen 1:4000 und 1:1. Dies hängt von der verwendeten Säure und deren Säurestärke, von der physiologisch wirksamen Konzentration des Alkaloids und vom Molekulargewicht von Alkaloid und Säure ab. Bei vielen Carbonsäuren, die eine vergleichsweise moderate Säurestärke haben, ist das Gewichtsverhältnis im Bereich von 1:96 bis 1:1.

Nach manchen Ausführungsformen liegt das Gewichtsverhältnis des zumindest einen Isochinolinalkaloids zu der zumindest einen Säure in der Zusammensetzung zwischen 1:96 und 1:30. Die zumindest eine Säure ist Äpfelsäure und das zumindest eine Isochinolinalkaloid ist Sanguinarin und/oder Chelerythrin.

Vorzugsweise handelt es sich bei der zumindest einen Säure um eine organische Säure, insbesondere eine Carbonsäure. Carbonsäuren haben sich als besonders gute Lösungsvermittler herausgestellt. Zudem haben sie den Vorteil, dass viele Carbonsäuren eine vergleichsweise moderate Säurestärke haben, leicht verfügbar und gesundheitlich unbedenklich sind.

Vorzugsweise ist die zumindest eine Säure wasserlöslich.

Vorzugsweise ist die zumindest eine Säure eine amphiphile organische Säure, insbesondere eine amphiphile Carbonsäure.

Nach Ausführungsformen ist die zumindest eine Säure eine amphiphile Säure, d.h., eine Säure, die sowohl wasserlöslich als auch in lipophiler Phase löslich ist.

Nach Ausführungsformen ist die zumindest eine Carbonsäure ausgewählt ist aus einer Gruppe umfassend: eine amphiphile Carbonsäure, eine aliphatische Carbonsäure, eine aromatische Carbonsäure, eine heterocyclische Carbonsäure, eine gesättigte Carbonsäure, eine ungesättigte Carbonsäure oder aus einer Kombination aus zwei oder mehr dieser Säuren.

Die zumindest eine Säure kann z.B. aus einer der folgenden Säuren oder aus einer Kombination von zwei oder mehreren dieser Säuren bestehen: Äpfelsäure, Milchsäure, Essigsäure, Zitronensäure, Ameisensäure, Sorbinsäure, Propionsäure, Askorbinsäure, Fumarsäure, Oxalsäure.

Vorzugsweise ist die zumindest eine Säure pulverförmig. Dies kann die Handhabung und das Abwiegen der ermittelten Menge an Säure erleichtern. Beispielsweise sind Äpfelsäure und Zitronensäure pulverförmig.

Im einfachsten Fall kann die Zusammensetzung nur aus dem zumindest einen Isochinolinalkaloid.

In anderen Ausführungsformen umfasst die Zusammensetzung neben dem zumindest einen Isochinolinalkaloid zumindest eine Säure und/oder Füllstoffe. Vorteilhafte weitere Füllstoffe sind Vitamine, Traubenzucker (Dextrose) und physiologische Säuren die der Körper benötigt wie Milchsäure. Vorteilhaft als weitere Komponente ist auch Sorbinsäure oder Kaliumsorbat da diese das Wachstum von Pilzen im Atemtrakt sicher verhüten.

Die Zusammensetzung kann um - vorzugsweise pH-neutrale - Füllstoffe angereichert sein, die die Dosierung der Zusammensetzung erleichtern und verhindern, dass eine zu große Menge Säure punktuell ins Lungengewebe gelangt. Beispielsweise kann eine solche Menge einer der hier beschriebenen Zusammensetzungen I-V verstäubt werden, sodass je nach Ziel der Anwendung zwischen 0,1 und 1000 mg Isochinolinalkaloid pro Quadratmeter Bodenfläche bzw. Objektfläche und Tag verstäubt werden.

Vorzugsweise wird die zumindest eine Säure schon bei der Herstellung der Zusammensetzung in Pulverform mit den anderen Komponenten der Zusammensetzung vermischt. Vorzugsweise wird zur Gewinnung einer pulverförmigen Zusammensetzung eine pulverförmige Säure wie z.B. Äpfelsäure oder Zitronensäure verwendet. Die Säure kann in der Lunge ein saures Milieu schaffen und die Alkaloide dadurch chemisch stabilisieren und in Lösung halten.

Nach manchen Ausführungsformen ist das zumindest eine Isochinolinalkaloid synthetischen Ursprungs.

Nach anderen Ausführungsformen wird das zumindest eine Isochinolinalkaloid der Zusammensetzung als Bestandteil von Pflanzenmaterial oder als Bestandteil eines Extrakts des Pflanzenmaterials bereitgestellt. Das Pflanzenmaterial oder das Extrakt kann das zumindest eine Isochinolinalkaloid zu einem Gewichtsanteil von 0,5%-99% enthalten. Insbesondere Extrakte oder synthetisch hergestellte Isochinolinalkaloide können nahezu vollständig aus Isochinolinalkaloiden bestehen und eine hohe chemische Reinheit aufweisen.

Das Pflanzenmaterial oder das Extrakt kann das zumindest eine Isochinolinalkaloid zu einem Gewichtsanteil von 0,1%-99%, vorzugsweise 5%-99% enthalten. Insbesondere Extrakte können über 10 Gew.% und nach manchen Ausführungsformen fast vollständig aus Isochinolinalkaloiden bestehen und eine hohe chemische Reinheit aufweisen.

Vorzugsweise enthält das Pflanzenmaterial das zumindest eine Isochinolinalkaloid zu einem Gewichtsanteil von über 0,01 %, vorzugsweise über 0,1%. Beispielsweise kann der Gehalt von Sanguinarin und Chelerythrin in manchen Pflanzenextrakten bei über 70% liegen. Ein hoher Alkaloidgehalt im Extrakt kann vorteilhaft sein, da dadurch die Einbringung nicht-löslicher oder bakteriell zersetzbarer organischer Substanzen in die Zusammensetzung und damit auch in die wässrige Lösung minimiert wird.

Die Verwendung von Extrakten (z.B. aus Ethanolextraktion) und synthetisch oder mikrobiologisch hergestellten Isochinolinalkaloiden kann gegenüber nur mechanisch aufgeschlossenem Pflanzenmaterial vorteilhaft sein, da der Isochinolinalkaloidgehalt der Extrakte bzw. synthetischen oder mikrobiologischen Produkte in der Regel höher ist als im pflanzlichen Gewebe und da sich das restliche Pflanzenmaterial nicht in Form von Sedimenten in der wässrigen Lösung absetzt. Dies kann Reinigungs- und Filterungsschritte bei der Herstellung der Dosierungseinheiten oder bei der Verabreichung von Tränkwasser oder einem flüssigen Aerosol, in welchem das zumindest eine Isochinolinalkaloid gelöst wurde, vermeiden helfen.

In einer erfindungsgemäßen Zusammensetzung können die Alkaloide in jedem beliebigen Verhältnis zueinander enthalten sein, also etwa im Bereich von Sanguinarin:Chelerythrin = 99%:1% bis 1%:99%. In einer speziellen Ausführungsform liegen die Alkaloide Sanguinarin und Chelerythrin in dem, auf dem natürlichen Verhältnis dieser Alkaloide im pflanzlichen Ausgangsmaterial beruhenden, Verhältnis 1:0,5 vor. Nach Ausführungsformen umfasst das Verfahren die Zugabe einer weiteren Substanz zu der Zusammensetzung. Die weitere Substanz kann z.B. ein Süßstoff, ein Zucker, eine Aminosäure oder eine Kombination von zwei oder mehr dieser Substanzen sein. Der Süßstoff kann z.B. Saccharin, Ca-Saccharin, Na-Saccharin, Neohesperidin-Dihydrochalcon (NHDC), Stervioside, Naringinsüßstoffe (z.B. Naringindihydrochalcone, auch als "Phloretin 4'-O-neohesperidosid" bekannt), Aspartam, ein Zuckeralkohol oder eine Kombination dieser Substanzen sein. Zu den verwendeten Zuckeralkoholen gehören insbesondere Sorbitol oder Xylit. Als Zucker kann beispielsweise Glucose oder Fructose verwendet werden. Die Verwendung von Sorbitol als einzigem Süßstoff der Zusammensetzung kann vorteilhaft sein, da es sehr schnell wasserlöslich ist, hohe Säuretoleranz hat und erheblich stabiler gegen mikrobiellen Verderb ist als die meisten Zucker wie z.B. Glucose. Die weitere Substanz kann auch aus ein oder mehreren Aromastoffen bestehen.

Die Verwendung der weiteren Substanzen, insbesondere von Süßstoffen, Zucker und/oder Aromen kann vorteilhaft sein, da diese die geschmackliche Wirkung der Säure kompensieren oder reduzieren können. Insbesondere durch die Beigabe von Süßungsmitteln kann verhindert werden, dass das Aerosol, sofern es eine organische Säure enthält, unangenehm schmeckt oder riecht. Die Verwendung von Sorbitol als einzigem Süßstoff und d Zusammensetzung kann vorteilhaft sein, da es sehr schnell wasserlöslich ist, hohe Säuretoleranz hat und im Gegensatz zu den meisten Zucker wie z.B. Glucose für diverse Bakterien, die an einer Sekundärinfektion beteiligt sein können, unverdaulich ist.

Die Gewichtsanteile der weitere(n) Substanz(en) und/oder der weiteren Zusatzstoffe sind also so zu wählen, dass der pH-Wert der alkaloidhaltigen wässrigen Lösung, die vernebelt und/oder zur Desinfektion von Objekten (z.B. als Immersionslösung oder als Wischlösung) genutzt wird, und die aus der Zusammensetzung hergestellt wird, trotz der weiteren Substanzen bei unter 6,8, vorzugsweise bei unter 6,5 liegt.

Nach Ausführungsformen werden der Zusammensetzung weitere Substanzen beigemengt, insbesondere Konservierungsmittel, Bindemittel und/oder Füllstoffe.

Beispielsweise können bei Verwendung von Extrakten geeigneter Pflanzen folgende Gewichtsverhältnissen bezüglich der Gesamtmenge an Isochinolinalkaloid in der Zusammensetzung auftreten:

| | |
|---|---|
| Sanguinarin | 35-90 Gew.% |
| Chelerythrin | 20-50 Gew.% |
| Chelirubin | 3 -5 Gew.% |
| Sanguirubin | 0 - 2 Gew.% |
| Chelilutin | 3 - 40 Gew.% |
| Sanguilutin | 0 - 15 Gew. % |
| Chelidonin | 0 - 10 % |
| Protopin | 0 - 30 % |
| Berberin | 0 - 10 % |
| Protoberberin | 0 - 10 % |
| α- Allocryptopin | 0 - 30 % |
| β- Allocryptopin | 0 - 10 % |

Die obigen Angaben entsprechen Durchschnittswerten, die in Abhängigkeit von der Pflanzensorte, dem Standort der Pflanze, dem Alter der Pflanze bei der Ernte und den verarbeiteten Pflanzenteilen (Wurzel, Blätter, etc.) schwanken können.

Die Zusammensetzung kann das zumindest eine Isochinolinalkaloid in Form eines physiologisch verträglichen Salzes enthalten, z.B. als Salz der Salzsäure (Chlorid), der Schwefelsäure (Sulfat) und der Tanninsäuren (Tannat).

Das Gewichtsverhältnis Alkaloid zu Säure beträgt nach Ausführungsformen 1:1000 bis 1:1, in manchen Ausführungsformen bei 1:600 bis 1:1, in manchen Ausführungsformen in einem Verhältnis von 1:96 bis 1:30. In Gewichtsverhältnisse von 1:1 können z.B. verwendet werden, wenn eine Säure mit sehr hoher Säurestärke verwendet wird (man von dieser also nur geringe Mengen benötigt) und eine vergleichsweise hohe physiologisch erforderliche Alkaloidkonzentration erwünscht ist. Höhere Anteile des Alkaloids an der Mischung können z.B. dann zum Einsatz kommen, wenn zum Zwecke einer Behandlung einer akuten Erkrankung kurzeitig besonders hohe Dosen des Alkaloids appliziert werden sollen und/oder wenn davon auszugehen ist, dass die Zusammensetzung in besonders weichem Wasser verdünnt werden soll, das nur eine geringe Pufferwirkung hat und daher schnell sehr sauer werden kann, sodass eine geringe Menge Säure ausreicht. Umgekehrt ist das Verhältnis von Alkaloid zu Säure eher im unteren Bereich der angegebenen Mengenverhältnisse (also wenig Alkaloid auf einen großen Gewichtsanteil Säure),wenn davon auszugehen ist, dass die Zusammensetzung - gelöst in einer geeigneten Menge Wasser- über einen längeren Zeitraum eher zum Zwecke der Krankheitsprophylaxe eingesetzt werden sollen und/oder wenn die Zusammensetzung zu einem späteren Zeitpunkt in besonders hartem Wasser verdünnt werden soll, das eine hohe Pufferwirkung hat und bei welchem daher höhere Mengen an Säure erforderlich sind um eine pH-Wert Absenkung auf unter 6.8, vorzugsweise auf unter 6.5 zu bewirken. Bei der Verwendung von Säuren mit geringer Säurestärke kommt eine größere Menge an Säure zum Einsatz.

### Aerosol aus flüssigen Schwebeteilchen

Nach Ausführungsformen besteht das Aerosol aus flüssigen Schwebeteilchen, die von einer Vernebelungsvorrichtung durch Vernebelung einer wässrigen Isochinolinalkaloidlösung erzeugt wird. Optional kann die wässrige Isochinolinalkaloidlösung weitere Substanzen beinhalten, z.B. eine Säure.

Die Verabreichung von Alkaloiden wie Sanguinarin oder Chelerythrin mittels eines Aerosols einer wässrigen isochinolinalkaloidhaltigen Lösung, die zudem eine Säure enthält, kann aus mehreren Gründen vorteilhaft sein: Zum Einen bewirkt die vorgenannte Kombination aus dem Alkaloid und der Säure eine chemische Stabilisierung des Alkaloids in seiner physiologisch wirksamen Form, zum anderen wirkt die Säure als Lösungsvermittler für das Alkaloid und verhindert dadurch ein Verstopfen der Düsen der Vernebelungsanlage. Der Zusatz der Säure kann also einen positiven synergistischen Effekt bewirken: Isochinolinalkaloide für sich alleine genommen lösen sich nicht oder zumindest nicht vollständig in Wasser, sondern bilden eine eigene lipophile Phase, bleiben an Gefäßwänden haften oder sedimentieren zusammen mit anderen lipophilen Substanzen. Die lipophile Phase kann Leitungen und Düsen verstopfen und stellt außerdem aus hygienischen Gründen ein Problem dar, da die lipophile Phase sich oftmals an der Oberfläche des Wassers, dem man Isochinolinalkaloide oder pflanzliche Extrakte mit Isochinolinalkaloiden beigegeben hat, absetzt und als Nährboden für unerwünschte Bakterien und Einzeller dient. Außerdem wurde beobachtet, dass Isochinolinalkaloide in wässriger Umgebung chemisch instabil sind, so dass nach dem Einbringen von Isochinolinalkaloiden in Wasser nach kurzer Zeit nicht mehr genügend Isochinolinalkaloide in der wässrigen Lösung vorhanden sind um eine physiologische Wirkung zu erzielen. Es ist bekannt, dass z.B. das Isochinolinalkaloid Sanguinarin in einer Umgebung mit einem Feuchtegehalt von über ca. 10 % einem raschen Abbau zum Sanguinarinhydrat (Dihydrosanguinarin) unterliegt, welches keine physiologische Wirksamkeit mehr hat. Je höher der Wassergehalt im Medium, desto rascher erfolgt der Wirkstoffabbau. Dagegen wurde beobachtet, dass die zumindest eine Säure (die aus einer einzigen oder einer Kombination mehrerer Säuren bestehen kann) überraschenderweise nicht nur eine Lösung des Alkaloids bzw. der Alkaloide in wässriger Lösung vermittelt, sondern darüber hinaus das Alkaloid bzw. Mischungen von Alkaloiden, insbesondere Sanguinarin, chemisch stabilisiert und vor einer Umwandlung in ein Hydrat (physiologisch inaktiv) schützt.

Der Begriff "wässrige Lösung eines Isochinolinalkaloids" oder "wässrige Isochinolinalkaloidlösung" oder "isochinolinalkaloidhaltige Lösung" ist hier nicht beschränkt auf eine homogene Verteilung einzelner Isochinolinalkaloidmoleküle in Wasser.

Vielmehr wird unter diesen Begriff auch eine Lösung verstanden, in welcher lipophile Stoffe wie z.B. viele Isochinolinalkaloide, in Form von Mizellen so in Wasser verteilt sind, dass eine Phasentrennung nicht beobachtbar ist. Insbesondere bedeutet "vollständig gelöst" hier ebenfalls, dass keine Sedimentation oder Ablagerung von Vesikeln einer Phase stattfindet und dass durch Filtration, z.B. mittels Papierfiltern, kein Herausfiltern der Isochinolinalkaloide oder deren Molekülverbände möglich ist.

Die isochinolinalkaloidhaltige wässrige Lösung, kann beispielsweise aus den hier beschriebenen Ausführungsformen der isochinolinalkaloidhaltigen Zusammensetzung, die selbst ein Pulver ist oder aus welcher sich das Pulver gewinnen lässt, wie folgt hergestellt werden:
Es wird gemäß Ausführungsformen eine Zusammensetzung aus zumindest einem Isochinolinalkaloid und zumindest einer Säure in einem bestimmten relativen Mengenverhältnis zueinander hergestellt wie bereits beschrieben. Das Gewichtsverhältnis der Säure und des Isochinolinalkaloids in der Zusammensetzung hängt von deren Säurestärke und der physiologisch wirksamen Isochinolinalkaloidkonzentration in der letztlich zu verabreichenden oder zu vernebelnden wässrigen Lösung ab, wobei die Menge und Art der Säure so gewählt ist, dass die besagte wässrige Lösung einen pH-Wert von unter 6,8, vorzugsweise 6,5 besitzt.

Beispielsweise kann die zumindest eine Säure flüssig sein. Dies ist z.B. bei der Propionsäure der Fall. Nach Ausführungsformen ist die Zusammensetzung flüssig. Sie kann also ein flüssiges Konzentrat sein, das dadurch gebildet wird, dass einer Zusammensetzung nach einer der vorab beschriebenen pulverförmigen Ausführungsformen noch eine geringe Menge Wasser zugegeben wird, um die pulverförmige Zusammensetzung in einer möglichst kleinvolumigen Menge Wasser aufzulösen. Beispielsweise können auf 5 g der pulverförmigen Zusammensetzung noch 5 g Wasser gegeben werden, um 10 g der flüssigen Zusammensetzung, auch "Konzentrat" genannt, herzustellen. Die 5 g Wasser können auch Dickungsmittel enthalten, um der resultierenden Zusammensetzung eine gelartige Konsistenz zu verleihen.

Die isochinolinalkaloidhaltige wässrige Lösung kann z.B. dadurch hergestellt werden, dass eine bestimmte Menge einer Zusammensetzung nach einer der oben beschriebenen Ausführungsformen in einer Menge Wasser, z.B. Leitungswasser, verdünnt wird. Dies kann z.B. dadurch erfolgen, dass die Menge der Zusammensetzung, z.B. eine oder mehrere Dosierungseinheiten in Kapselform, Pulverform, Gelform, Tablettenform oder als Konzentrat dem Leitungswasser beigegeben wird und für mehrere Minuten das Wasser mit der Zusammensetzung bewegt wird, z.B. durch eine Rühr- oder Schüttel- oder Pumpapparat oder durch manuelle Bewegungen. Die beigegebene Menge der Zusammensetzung ist so bemessen, dass die resultierende isochinolinalkaloidhaltige wässrige Lösung eine physiologisch wirksame Konzentration des zumindest einen Isochinolinalkaloids enthält. Nach einigen Minuten löst sich die Zusammensetzung vollständig in dem Wasser auf und die wässrige Isochinolinalkaloidlösung entsteht.

### Kurzbeschreibung der Figuren

Exemplarische Ausführungsformen der Erfindung sind in den anhängenden Zeichnungen und Tabellen veranschaulicht. Darin zeigen:
- Figur 1: ein Blockdiagramm eines Gebäudes mit Vernebelungsvorrichtung,
- Figur 2: ein Blockdiagramm eines Fahrzeugs mit Vernebelungsvorrichtung,
- Figur 3: ein Flussdiagramm einer Ausführungsform eines erfindungsgemäßen Verfahrens,
- Figur 4: eine Gesichtsmaske,
- Figur 5: einen Querschnitt einer einseitig behandelten Gesichtsmaske,
- Figur 6: einen Querschnitt einer zweiseitig behandelten Gesichtsmaske,
- Figur 7: einen Querschnitt einer behandelten Tischplatte, und
- Figur 8: ein Nasenspray gegen Covid-19.

### Ausführliche Beschreibung

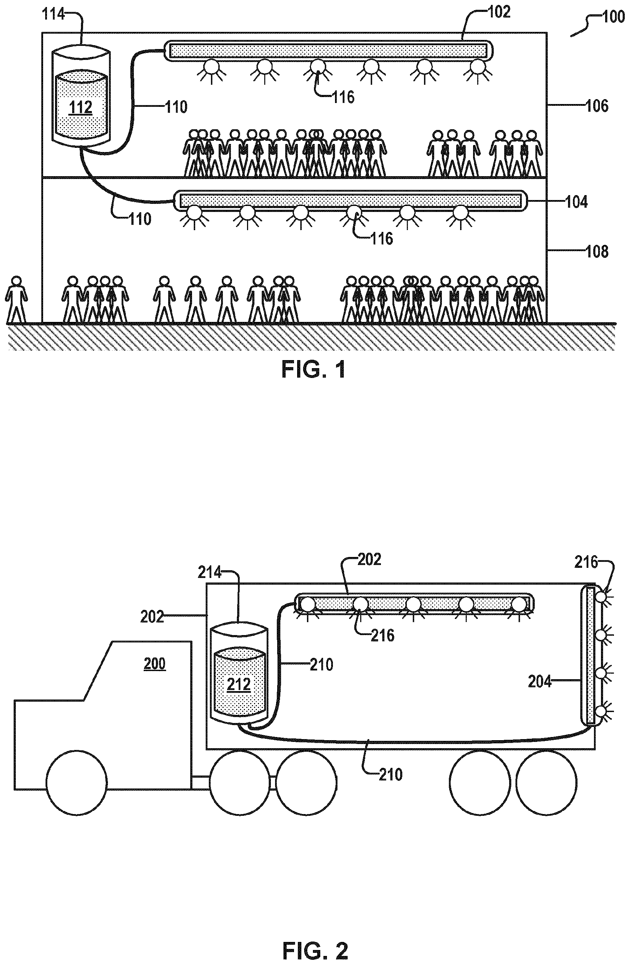

Figur 1 zeigt ein Blockdiagramm eines Gebäudes 100 mit Vernebelungsvorrichtung. Das hier dargestellte Gebäude umfasst zwei Stockwerke 106, 108. In jedem Stockwerk sind mehrere Vernebelungsdüsen 116 angebracht, die über Leitungen 110 mit einem oder mehreren Behältern 114, die z.B. als Wassertank ausgebildet sein können, verbunden. Der Behälter 114 enthält eine wässrige Lösung mit mindestens einem Isochinolinalkaloid, z.B. einer Mischung aus etwa gleichen Anteilen an Sanguinarin und Chelerythrin oder Sanguinarin und Berberin. Die wässrige Alkaloidlösung 112 wird über die Leitungen 110 zu den einzelnen Düsen 116 geleitet. Beispielsweise kann der Behälter samt dem Leitungssystem mit Druck beaufschlagt werden, um die wässrige Lösung durch die Düsen zu treiben. Es ist aber auch möglich, dass die Düsen 116 individuell oder gruppenweise, z.B. pro Raum, mit einem Druck beaufschlagt werden können. Letzteres hat den Vorteil, dass die Menge des vernebelten Aerosols individuell pro Düse oder pro Raum eingestellt werden kann. Beispielsweise zeigt Figur 1 eine erste Gruppe 104 von Düsen, die ein Aerosol für das erste Stockwerk erzeugen, und eine zweite Gruppe 102 von Düsen, die ein Aerosol für das zweite Stockwerk erzeugen, wobei die beiden Gruppen unabhängig voneinander reguliert werden können.

Die Vernebelungsanlage kann aus einem oder mehreren mit einander verbundenen Modulen bestehen. Sie ist vorzugsweise an der Decke oder an den oberen Bereichen der Seitenwände einzelner Räume angebracht. Insbesondere kann es sich bei dem Raum um einen Aufzug oder ein Treppenhaus handeln. Hier ist die Kontaktdichte zwischen Menschen und damit auch die Ansteckungsgefahr mit Covid-19 besonders hoch. Dadurch, dass die Vernebelung oder Verstäubung der isochinolinhaltigen Zusammensetzung zumindest im Treppenhaus und/oder Aufzug vorgenommen wird, kann es möglich sein, zu verhindern, dass insbesondere mehrstöckige Gebäude, z.B. Hochhäuser, als Büroräume wegen der großen Ansteckungsgefahr nicht mehr nutzbar sind bzw. dass sich Menschen, die in Hochhäusern wohnen, im Treppenhaus oder dem Aufzug anstecken.

Figur 1 zeigt schematisch eine Vernebelungsanlage für den Innenbereich eines Gebäudes. In analoger Weise können jedoch auch Verstäubungsanlagen installiert werden, die Festkörperaerosole generieren, und/oder Vernebelungs- oder Verstäubungsanlagen für den Außenbereich. Letzteres ist z.B. vorteilhaft für die Außenanlagen von Cafes und Restaurants, für Sportstadien, Freizeitparks, Zoos oder sonstiger Bereiche außerhalb von Gebäuden, die von vielen Menschen frequentiert werden.

**Figur 2** zeigt ein Blockdiagramm eines Fahrzeugs 200 mit einer Vernebelungsvorrichtung. Das Fahrzeug kann z.B. zur großflächigen Desinfektion von Straßen, Lagerhallen, Sportstadien oder Freizeitparks genutzt werden. Es enthält mindestens ein Behältnis 214 mit einer Zusammensetzung 212 mit mindestens einem Isochinolinalkaloid, z.B. einer wässrigen Lösung mit dem oder den Alkaloiden und optional einer organischen Säure als Lösungsvermittler und Stabilisator. Der Behälter ist über Leitungen 210 mit zwei Vernebelungseinheiten 202, 204 verbunden. Jede der Vernebelungseinheiten umfasst eine oder (hier) mehrere Düsen 216. Die Düsen oder zumindest die Öffnungen der Düsen befinden sich außerhalb des Fahrzeuges. Beispielsweise dienen die Düsen der Einheit 204 dazu, den Boden und sonstige Objekte hinter dem Fahrzeug zu desinfizieren. Die Einheit 202 dient dazu, Objekte zur Linken (aus Sicht in Fahrtrichtung) des Fahrzeugs zu desinfizieren. Das Fahrzeug kann eine weitere Düseneinheit auf der anderen Fahrzeugseite haben (nicht gezeigt), die dazu dient, Objekte zur Rechten des Fahrzeugs zu desinfizieren. Vorzugsweise sind die Düsen oder zumindest die Düseneinheiten regulierbar bezüglich der Sprührichtung und/oder der Menge der pro Zeit vernebelten Zusammensetzung. Dies ermöglicht es, die Desinfektion zielgerichtet dem Gelände und dem benötigten Sicherheitsniveau anzupassen.

In analoger Weise können auch Verstäubungsanlagen auf Fahrzeugen installiert werden, die Festkörperaerosole generieren.

Die Vernebelungs- oder Verstäubungsanlage kann auch als für einen Menschen portables System ausgebildet sein, das Tragegurte wie bei einem Rucksack aufweist, sodass eine Person sich die Vorrichtung z.B. auf den Rücken schnallen kann. Diese Ausführungsvariante ist vorteilhaft für Gelände, auf denen ein Fahrzeug nicht fahren kann, wie z.B. sehr kleine Gassen oder die Sitzreihen in großen Sportstadien.

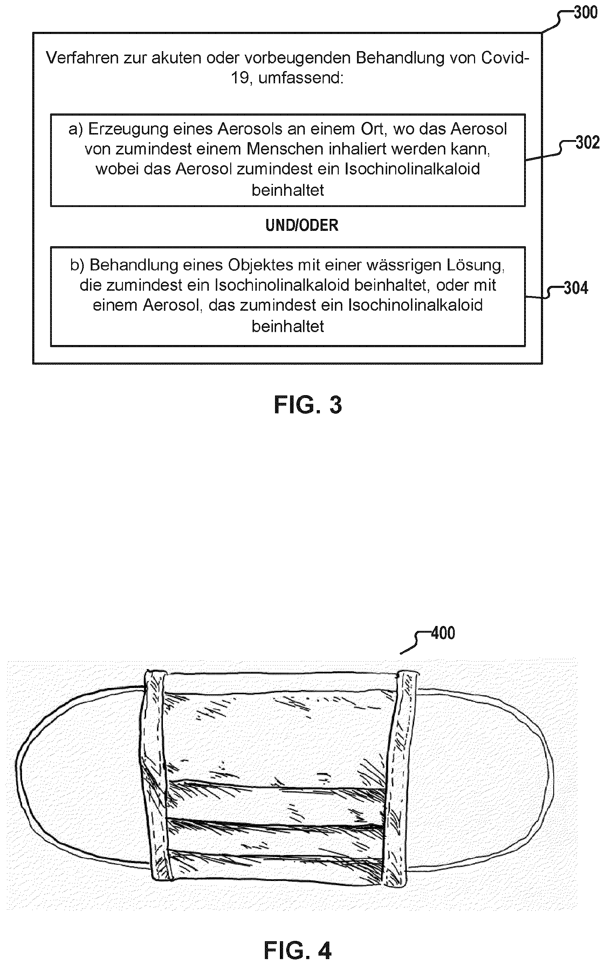

**Figur 3** zeigt ein Flussdiagramm gemäß Ausführungsformen eines erfindungsgemäßen Verfahrens 300.

In einer Ausführungsvariante a) wird ein Aerosol an einem Ort erzeugt, wo das Aerosol von zumindest einem Menschen inhaliert werden kann. Das Aerosol besteht aus flüssigen oder festen Schwebeteilchen, die zumindest ein Isochinolinalkaloid beinhalten. Beispielsweise wird das Aerosol von einem Nasenspray, Rachenspray oder medizinischen Inhalator erzeugt, wobei das Aerosol in der Nase, im Mund- oder Rachenraum oder in einem Abstand vom Gesicht einer Person von weniger als 15 cm erzeugt. Hierbei ist es möglich, einer Person gezielt eine vergleichsweise hohe Dosis an Isochinolinalkaloiden zuzuführen. Diese Verfahrensvarianten können z.B. zur Akutbehandlung einer Covid-19 Erkrankung oder einer Erkrankung, deren Symptome zumindest auf Covid-19 schließen lässt, verwendet werden.

In einer anderen Ausführungsvariante b) wird nicht eine Person, sondern ein Objekt mit einer wässrigen Lösung, die zumindest ein Isochinolinalkaloid beinhaltet, oder mit einem Aerosol, das zumindest ein Isochinolinalkaloid beinhaltet, behandelt. Das Objekt ist ein Gerät, Kleidungsstück oder Gegenstand ist, das für den Kontakt mit der menschlichen Haut bestimmt ist. Das Objekt kann gezielt individuell oder Als Mitglied einer Gruppe typgleicher Objekte behandelt werden, z.B. indem ein bestimmter Stuhl oder eine Tischplatte mit der Lösung oder dem Aerosol behandelt wird, oder indem man Gesichtsmaksten während des Herstellungsprozesses oder nach Gebrauch durch das Klinikpersonal zur erneuten Verwendung desinfiziert. Das Objekt kann aber auch im Zuge einer großflächigen Desinfektion von Straßen, Räumen oder Gebäuden zusammen mit anderen Objekten, die nicht für den Kontakt mit der menschlichen Haut bestimmt sind, behandelt werden (z.B. Stühle eines Restaurants, die am Straßenrand stehen).

Unter einem Objekt, das für den Kontakt mit der menschlichen Haut bestimmt ist, wird hier jegliches Objekt verstanden, das bei normalem, bestimmungsgemäßen Gebrauch berührt werden muss. Hierzu gehören z.B. Stühle, Tische, und sonstige mobile Gegenstände. Insbesondere gehören hierzu Objekte, die bei bestimmungsgemäßen Gebrauch mit dem Gesicht einer Person in Kontakt kommen (z.B. Gesichtsmasken, Brillen, medizinische Geräte für den Mund-Nasen-Rachenbereich etc) und/oder die über einen längeren Zeitraum von mindestens mehreren Stunden als Kleidungsstück oder Accessoire getragen werden, z.B. medizinische Schutzanzüge, Verbände, etc.

Auch Mischformen und Kombinationen von a) und b) sind möglich: Beispielsweise können isochinolinalkaloidhaltige Raumsprays und Desinfektionsmittel, die großflächig in der Luft innerhalb oder außerhalb eines Gebäudes verteilt werden, sowohl von Personen inhaliert werden und hierbei prophylaktisch oder therapeutisch wirken, als auch sich auf der Oberfläche von Objekten wie Stühlen, Kleidungsstücken oder Tischen absetzen und dadurch diese Oberflächen desinfizieren.

**Figur 4** zeigt eine medizinische Gesichtsmaske 400. Durch einseitiges oder beidseitiges Besprühen der Maske mit einem isochinolinalkaloidhaltigen Aerosol, insbesondere einem Tröpfchenaerosol, oder durch Immersion der gesamten Maske in eine isochinolinalkaloidhaltige Lösung kann das Risiko der Schmierinfektion durch falsche Handhabung der Maske und die Gefahr der Verkeimung bei zu langem Gebrauch reduziert werden.

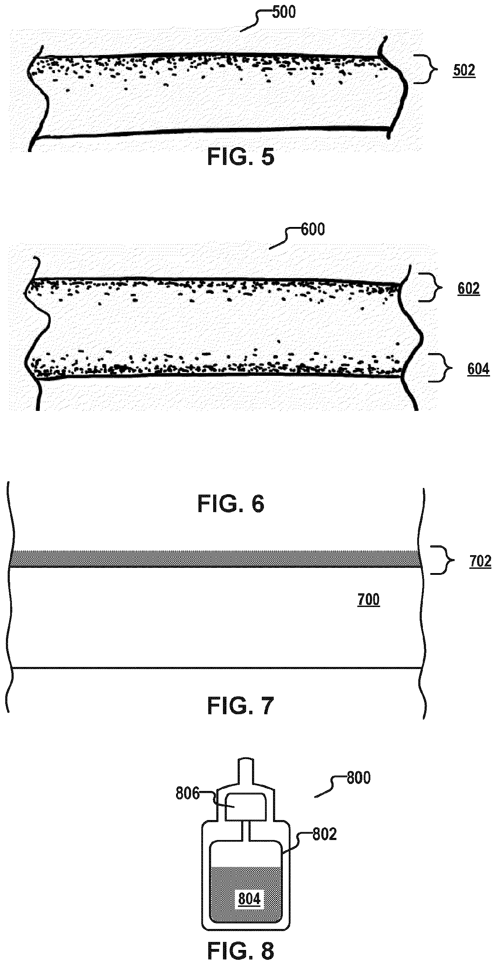

**Figur 5** zeigt einen Querschnitt einer Gesichtsmaske 500, die einseitig mit einer isochinolinalkaloidhaltigen Zusammensetzung behandelt wurde. Die Zusammensetzung ist dabei bis zu einer gewissen Tiefe in das Material eingedrungen und bildet eine isochinolinalkaloidhaltige Schicht 502, die SARS-CoV-2 Viren abtöten kann.

**Figur 6** zeigt einen Querschnitt einer Gesichtsmaske 600, die beidseitig mit einer isochinolinalkaloidhaltigen Zusammensetzung behandelt wurde. Die Zusammensetzung ist dabei bis zu einer gewissen Tiefe von beiden Seiten in das Material eingedrungen und bildet zwei isochinolinalkaloidhaltige Schichten 602, 604, die SARS-CoV-2 Viren abtöten können. Es ist auch möglich, die Maske in einer isochinolinalkaloidhaltigen Lösung zu tränken, sodass das gesamte Maskenmaterial das zumindest eine Isochinolinalkaloid enthält.

**Figur 7** zeigt den Querschnitt einer Tischplatte 700. Die Tischplatte ist ein weiteres Beispiel für ein Objekt, das mit einer isochinolinalkaloidhaltigen Zusammensetzung behandelt wurde. Da das Material nicht porös ist, haftet die isochinolinalkaloidhaltige Zusammensetzung an der Oberfläche der Tischplatte und bildet eine dünne Schicht 702 aus, die vor SARS-COV-2 und anderen Viren schützt.

**Figur 8** zeigt ein Nasenspray 800 für die akute oder prophylaktische Behandlung von Covid-19. Das Nasensprach beinhaltet ein Behältnis 802, das mit einer isochinolinalkaloidhaltigen Zusammensetzung 804 gefüllt ist, und eine Vernebelungseinheit 806, z.B. eine Drucksprüheinheit, besitzt. Das Nasenspray ist so geformt, dass das von der Sprüheinheit gebildete Aerosol direkt in die Nase appliziert werden kann.

### Bezugszeichenliste

- 100: Gebäude
- 102: Einheit mit Düsen für ein oberes Stockwerk
- 104: Einheit mit Düsen für das Erdgeschoß
- 106: erstes Stockwerk
- 108: Erdgeschoß
- 110: Leitungen
- 112: Zusammensetzung
- 114: Behältnis
- 216: Düsen
- 200: Fahrzeug
- 202: Einheit mit Düsen für linke Seite
- 204: Einheit mit Düsen für Fahrzeugrückseite
- 210: Leitungen
- 212: Zusammensetzung
- 214: Behältnis
- 216: Düsen
- 300: Verfahren
- 302-304: Verfahrensschritte
- 400: medizinische Gesichtsmaske
- 500: Querschnitt medizinischer Gesichtsmaske
- 502: isochinolinalkaloidhaltige Schicht innerhalb der Maske
- 600: Querschnitt medizinischer Gesichtsmaske
- 602: isochinolinalkaloidhaltige Schicht innerhalb der Maske
- 604: isochinolinalkaloidhaltige Schicht innerhalb der Maske
- 700: Querschnitt Tischplatte
- 702: isochinolinalkaloidhaltige Schicht auf der Tischplatte
- 800: Nasenspray
- 802: Behältnis
- 804: isochinolinalkaloidhaltige Zusammensetzung
- 806: Vernebelungseinheit

## Patentansprüche

1. Verfahren (300) zur akuten oder vorbeugenden Behandlung von Covid-19, beinhaltend:
- a) Erzeugung (302) eines Aerosols an einem Ort, wo das Aerosol von zumindest einem Menschen inhaliert werden kann, wobei das Aerosol aus Schwebeteilchen besteht, die zumindest ein Isochinolinalkaloid beinhalten; und/oder
- b) Behandlung (304) eines Objektes (400, 500, 600, 700) mit einer wässrigen Lösung, die zumindest ein Isochinolinalkaloid beinhaltet, oder mit einem Aerosol, das zumindest ein Isochinolinalkaloid beinhaltet, wobei das Objekt ein Gerät, Kleidungsstück oder Gegenstand ist, das für den Kontakt mit der menschlichen Haut bestimmt ist.

2. Verfahren nach Anspruch 1a), wobei das Aerosol von einem medizinischen Zerstäuber, insbesondere einem portablen Zerstäuber, insbesondere einer Nasensprayflasche oder einem Rachensprayflasche mit Zerstäuberaufsatz erzeugt wird.

3. Verfahren nach einem der vorhergehenden Ansprüche 1a) oder 2, wobei die Konzentration des zumindest einen Isochinolinalkaloids in dem Aerosol und die Menge des vernebelten oder versprühten Aerosols so eingestellt sind, dass mindestens 0,1 mg, insbesondere mindestens 0,5 mg, vorzugsweise mindestens 1 mg des zumindest einen Isochinolinalkaloids pro Tag und Person inhaliert werden.

4. Verfahren nach Anspruch 1b), wobei das Objekt ausgewählt ist aus einer Gruppe umfassend:
- Medizinische Gesichtsschutzmasken, insbesondere FFP2 und FFP3 Masken;
- Operations-Masken und Alltags-Gesichtsmasken mit und ohne Filtereinsatz;
- Sitze, Haltestangen, Haltegriffe und sonstige Vorrichtungen innerhalb von Land-, Luft- und Wasserfahrzeugen, insbesondere von Bussen, Zügen, Flugzeugen und Passagierschiffen;
- Mobiliar von Gebäuden, insbesondere von Restaurants, Schulen, Kindergärten, Heimen, Firmen und Behörden sowie Hallen und Säle für Großveranstaltungen;
- Betttücher oder Handtücher;
- Türgriffe;
- Medizinische Verbände;
- Medizinische Einwegartikel, insbesondere Schutzkleidung.

5. Verfahren nach Anspruch 1b) oder 3, wobei die Behandlung aus einem Tränken des Objekts mit der wässrigen Lösung, eine Immersion des Objekts in der wässrigen Lösung oder ein Abwischen des Objekts mit der wässrigen Lösung umfasst.

6. Verfahren nach einem der Ansprüche 1b, 3, 4, oder 5, wobei bei der Aerosolerzeugung die Konzentration des zumindest einen Isochinolinalkaloids in dem Aerosol und die Menge des vernebelten oder versprühten Aerosols so eingestellt sind, dass mindestens 0,1 mg des zumindest einen Isochinolinalkaloids pro Quadratmeter Bodenfläche und pro Tag oder pro Quadratmeter Objekt und Tag verstäubt werden.

7. Verfahren nach Anspruch 6, wobei die Konzentration des zumindest einen Isochinolinalkaloids in dem Aerosol und die Menge des vernebelten oder versprühten Aerosols so eingestellt sind, dass weniger als 2g, insbesondere weniger als 1 mg des zumindest einen Isochinolinalkaloids pro Quadratmeter Bodenfläche und pro Tag oder pro Quadratmeter Objektoberfläche und Tag verstäubt werden.

8. Verfahren nach einem der vorigen Ansprüche, wobei das Aerosol von einer Vorrichtung zur Belüftung von Gebäuden oder Gebäudeteilen, insbesondere von einer Klimaanlage oder Luftfilteranlage, erzeugt wird.

9. Verfahren nach einem der vorigen Ansprüche, wobei das Aerosol von einer Aerosolerzeugungseinheit erzeugt wird, welche für die Installation auf einem Fahrzeug (200) oder für den Transport durch eine Person bestimmt ist, wobei die Aerosolerzeugungseinheit beinhaltet:
- einen Tank (214) mit einer Zusammensetzung mit dem zumindest einen Isochinolinalkaloid;
- eine Düse (216), wobei die Düse mit dem Tank leitend verbunden ist und dazu ausgebildet ist, die Zusammensetzung in ein Aerosol zu zerstäuben oder zu vernebeln; und
- eine Druckerzeugungseinheit, die dazu ausgebildet ist, die Zusammensetzung oder das Aerosol mit einem Druck zu beaufschlagen, der hinreichend ist, um das Aerosol in einem Radius von mindestens 0,5 m, vorzugsweise mindestens 1 m um die Düse herum in mindestens eine Richtung zu verbreiten.

10. Verfahren nach einem der vorhergehenden Ansprüche, wobei es sich bei dem zumindest einen Isochinolinalkaloid um Sanguinarin, Chelerythrin, Chelirubin, Chelidonin, Chelilutin, Cholerythrin, Berberin, Protoberberin, Protopin, α- Allocryptopin, β-Allocryptopin, Macarpin, Cularin oder eine Kombination von zwei oder mehr der vorgenannten Isochinolinalkaloide handelt.

11. Verfahren nach einem der vorhergehenden Ansprüche, wobei mindestens 10%, vorzugsweise mindestens 30% des zumindest einen Isochinolinalkaloids aus Sanguinarin, Berberin und/oder Chelerythrin bestehen.

12. Verfahren nach einem der vorhergehenden Ansprüche,
- wobei das Aerosol aus flüssigen Schwebeteilchen besteht, die von einer Vernebelungsanlage durch Vernebelung einer wässrigen Isochinolinalkaloidlösung erzeugt wird; oder
- wobei das Aerosol ein Feststoffaerosol ist, das insbesondere durch Verstäubung des zumindest einen Isochinolinalkaloids oder dessen Salzes erzeugt wird.

13. Zusammensetzung (112, 212, 804) zur akuten oder vorbeugenden Behandlung von Covid-19, beinhaltend zumindest ein Isochinolinalkaloid.

14. Zusammensetzung nach Anspruch 13, wobei die Zusammensetzung ausgebildet ist als:
- wässrige Lösung, die das zumindest eine Isochinolinalkaloid beinhaltet;
- Pulver, das das zumindest eine Isochinolinalkaloid beinhaltet;
- Aerosol aus Pulverpartikeln oder Flüssigkeitströpfchen, wobei das Aerosol zumindest ein Isochinolinalkaloid beinhaltet.

15. Objekt (400, 500, 600, 700), wobei das Objekt ein Gerät, Kleidungsstück oder Gegenstand ist, wobei das Objekt für den Kontakt mit der menschlichen Haut bestimmt ist und wobei das Objekt zur Verhinderung einer Ansteckung mit Covid-19 zumindest ein Isochinolinalkaloid beinhaltet oder trägt.

16. Objekt nach Anspruch 15, ausgewählt aus einer Gruppe umfassend:
- medizinische Gesichtsschutzmasken, insbesondere FFP1, FFP2 und FFP3 Masken;
- Operations-Masken, Alltagsgesichtsmasken und Mundschutzmasken mit und ohne Filtereinsatz;
- Betttücher oder Handtücher;
- medizinische Verbände;
- medizinische Einwegartikel, insbesondere Schutzkleidung.

17. Nasenspray (800), Rachenspray oder Raumspray zur Verhinderung einer Ansteckung mit oder zur Behandlung von Covid-19, beinhaltend:
- ein portables Behältnis (802) mit einer Zusammensetzung (804) mit zumindest einem Isochinolinalkaloid, und
- einer Einheit (806) zur Erzeugung eines Aerosols aus der Zusammensetzung.

18. Vorrichtung zur Erzeugung eines Aerosols im Außenbereich oder Innenbereich von Gebäuden (100), insbesondere von Fahrstühlen, wobei die Vorrichtung umfasst:
- ein Behältnis (114) mit einer Zusammensetzung (112) mit zumindest einem Isochinolinalkaloid, und
- einer Einheit (116) zur Erzeugung eines Aerosols aus der Zusammensetzung.
